# EUROPEAN PATENT APPLICATION

(11) **EP 4 635 472 A1**
(43) Date of publication of application: **22.10.2025**
(21) Application number: 25170863.2
(22) Date of filing: 16.04.2025
(51) Int. Cl.: A61K 8/39, A61Q 19/00, A61K 8/67, A61K 8/92, A61K 8/9789

(54) **COSMETIC COMPOSITION COMPRISING AN EXTRACT OF FRAXINUS EXCELSIOR ASH BARK SOLUBILIZED IN JUGLANS REGIA WALNUT SEED OIL AND POLYGLYCERYL-4 OLEATE AND TOCOPHEROL**

(30) Priority: 17.04.2024 LU 506955
(71) Applicant: Société De Recherche Cosmétique S.à.r.L., 2314 Luxembourg (LU)
(72) Inventor: DUCHATEAU, Maëva, 57100 Thionville (FR); MERINVILLE, Eve, 43123 PARMA (IT)
(74) Representative: Dennemeyer & Associates S.A.

(57) **Abstract**

The present invention concerns an intermediary cosmetic composition, characterized in that it comprises:
- (a1) 1 to 30 wt% of an extract of Fraxinus Excelsior Ash bark, and
- (b1) 60 to 80 wt% of Juglans Regia walnut seed oil, and
- (c1) 5 to 30 wt% of polyglyceryl-4 oleate, and
- (d1) 0,1 to 1 wt% of Tocopherol.

It also concerns a final cosmetic product composition and a non-therapeutic cosmetic process for skin care and a non-therapeutic use of the final cosmetic product for topical application.

## Description

### Technical field:

The present invention relates to the cosmetic field namely compositions for topical application, for cosmetically protecting the human skin against environmental aggressions, preventing, delaying and/or fighting the signs of skin aging, improving the skin barrier, improving hydration.

### State of the art:

MINTEL, 27 February 2023 entitled "Gleaming Brightening Activating Eye Cream" discloses an eye cream that claims to moisturize, nourish, increase firmness and elasticity of the skin, improving sagging. It includes Fraxinus excelsior bark extract and juglans regia seed oil. It also includes two other ingredients: polyglyceryl-6-distearate and polyglyceryl-3-beeswax.

CN106619322 discloses a pair of eye masks with a function of removing eye bags, wherein the eye bags removal composition is prepared from a Fraxinus excelsior bark extract and siloxanetriol alginate; a water-containing carrier. Eye masks with the function of removing eye bags can promote blood circulation, enhance metabolism, promote lipolysis and protect and reinforce capillaries, and has multiple eye bag removal effects.

RO126369 provides a pulverulent phytotherapeutic product for the treatment of hepatitis, migraines and asthenia and comprises 10-14 parts of Fraxinus excelsior bark extract and 4 parts of Juglans Regia.

RO 127268 provides a phytotherapeutic product for the elimination of stress, migraines and asthenia and comprises 14 parts of Fraxinus excelsior bark extract and 4 parts of Juglans Regia.

Kostova et al relates to « chemical components of Fraxinus species", Fitoterapia, IDB Holding, Milan, Italy, vol.78, no 2, February 1, 2007.

Whang W. K. et al relates to « natural compounds, fraxin and chemicals structurally related to fraxin protect cell from oxidative stress". Experimental and molecular medicine, Seoul, Korea, vol.37, no 5, 31 october 2005.

CN106074320 provides a facial cream containing 0.1%-5.0% of Fraxinus excelsior bark extract, and has the effect of maintaining moisture and moisturizing and regaining the moisture of skin.

CN114699345 provides an application of a plant extract in whitening and spot-fading cosmetics and relates to the technical field of cosmetics and medical treatment and comprises juglans regia leaves.

US2024/074964 provides a nutraceutical formulation which could be used for holistic skin care and treatment purposes and comprises juglans regia extract. It teaches about the inclusion of Juglans regia extract in an amount between 2 to 27,5 wt%.

Joana S. et al related to "vitamin E composition of walnuts (Juglans regia L.): a 3-year comparative study of different cultivars - Pubmed", Journal of Agricultural and Food chemistry, vol.53, no 13, 1 June 2005.

CN105853315 provides a face cream containing green tea essence and 5 to 8 wt% of European ash tree bark extract.

Other prior art documents are FR3003170**,** FR3012963**,** FR3011181**,** FR3011182 **and** FR3011183**.**

### The following compounds are all well-known separately by a person skilled in the art:

### An extract of Fraxinus Excelsior Bark:

Fraxinus Excelsior Bark Extract, also known as **European Ash Bark Extract,** is a natural ingredient derived from the bark of the European Ash tree (Fraxinus excelsior L., Oleaceae). In scientific literature, this extract is described to have anti-inflammatory and anti-microbial properties. In a cosmetic context, this extract serves as a skin conditioning agent, which means it helps to maintain and improve the skin's appearance, texture, and overall health.

### Juglans Regia seed oil:

Walnut oil (also known as juglans regia seed oil) is an oil derived from the meats of walnuts. Walnuts are widely recognized as one of the healthiest nuts, and some studies suggest that even just ingesting walnut oil can promote better skin health. Walnut oil is an emollient, skin-conditioning agent first and foremost, meaning it has a softening, soothing effect on the skin. Studies suggest that it promotes better skin health, helping heal wounds faster and treat some skin conditions, like eczema. Known main benefits: hydrates and strengthens the skin's surface, prevents water loss, defends against environmental stress, reduces signs of aging, and eliminates flaky, dehydrated skin.

### Polyglyceryl-4 oleate (also known as PGMO4):

Polyglyceryl-4 oleate is a surfactant mainly used as an emulsifier in the cosmetic industry; it brings oil and water together. It can be used to emulsify water-in-oil creams and lotions and can be included in anhydrous formulations to create products that will self-emulsify when mixed with water. The Hydrophilic-Lipophilic Balance (HLB) is a system used to describe the properties of surfactants, particularly in the cosmetic industry. Developed by William C. Griffin in the late 1940s, the HLB system helps formulators to select the best surfactants for creating stable emulsions. The HLB value ranges from 0 to 20. Lower values (0-6) indicate lipophilic (oil-loving) properties, while higher values (8-20) indicate hydrophilic (water-loving) properties. With an HLB of 4, Polyglyceryl-4 oleate has physico-chemical properties that makes it soluble in oils, and suitable to help extracting hydrophilic molecules toward the oil. Polyglyceryl-4 oleate is a cloudy viscous liquid at room temperature.

### Tocopherol:

Tocopherol, also known as vitamin E, is a natural ingredient found in vegetables oils such as wheatgerm oil, olive oil and sunflower oil. Tocopherol is widely used in the cosmetic industry to protect vegetable oils and cosmetic compositions toward rancidity, to improve their shelf life by preventing the oxidation of fatty acids.

### Summary of the invention:

The present invention is set out in the appended set of claims.

### Problem solution approach:

- The **closest prior art** is MINTEL, 27 February 2023 entitled "Gleaming Brightening Activating Eye Cream" because it concerns anti-aging properties of an eye cream. It includes Fraxinus excelsior bark extract and juglans regia seed extract. It also includes other ingredients like polyglyceryl-6-distearate and polyglyceryl-3-beeswax, which are not part of the present invention.
- The **difference** between the present invention and the closest prior art "Gleaming Brightening Activating Eye Cream" is the amount of the ingredients in the intermediate cosmetic composition of the present invention, i.e.:
   - 1 to 30 wt% of an extract of Fraxinus Excelsior Ash bark, and
   - 60 to 80 wt% of Juglans Regia walnut seed oil, and
   - 5 to 30 wt% of polyglyceryl-4 oleate, and
   - 0,1 to 1 wt% of Tocopherol
   and also the amount of the ingredients in the final cosmetic product composition, i.e.:
   - (a5) 0.09 to 0.13 wt% of an extract of Fraxinus Excelsior Ash bark,
   - (b5) 0.69 to 0.73 wt% of Juglans Regia walnut seed oil,
   - (c5) 0.16 to 0.20 wt% of polyglyceryl-4 oleate,
   - (d5) 0.001 to 0.73 wt% of tocopherol,
   - (e5) the rest being one or several other ingredients (or a cosmetical acceptable carrier).
- The **technical effect** of the difference is that the cosmetic composition provides improved anti-aging properties, with a reduction of the carbonylation of skin proteins, improvement and restoration of the skin barrier, hydration of the skin, ensuring a certain cohesion and a strengthening of the dermo-epidermal junction and the extracellular matrix, anti-inflammatory benefits, antioxidant and detoxification benefits, anti-microbial benefits and anti-pigmentation properties due to the total content in polyphenols (mg/kg eq. Oleuropein) +/- 20% (n=2) being 5900 when using Polyglyceryl-4 oleate, while it is much less than 2970 when using polyglyceryl-6-distearate (see table 5).
- The objective **technical problem** to be solved by the present invention is to provide an additional (improved) anti-aging cosmetic composition, and to reduce the carbonylation of skin proteins, and to improve and to restore the skin barrier, hydration of the skin, to ensure a certain cohesion and a strengthening of the dermo-epidermal junction and the extracellular matrix, anti-inflammatory benefits, anti-oxidant and detoxification benefits, anti-microbial benefits and anti-pigmentation properties.
- A person skilled in the art would not be led to use the amounts of the intermediary cosmetic composition of the present invention.
- A person skilled in the art would not be led to replace polyglyceryl-4 oleate by polyglyceryl-6-distearate because polyglyceryl-6-distearate is partially soluble in oil and because polyglyceryl-6-distearate does not allow to obtain an acceptable polyphenols content (see table 5).
- A person skilled in the art would not be led to replace Juglans Regia seed oil **with** an extract of Juglans Regia seed because the extraction solvent must necessarily be a fatty substance. This fatty substance is preferably of vegetable origin, chosen from glyceridic fatty substances, preferably a vegetable oil, non-glyceridic fatty substances, preferably natural wax, or a mixture, as described in the OLEOS patent (FR2943684). Juglans Regia seed oil was chosen due to these criteria and to get an optimal oleo-extraction process. Juglans Regia seed oil was used for this invention as co-solvent and due to these cosmetic properties (moisturizing and nourishing).
- None of the prior art discloses the amounts of the claimed composition of the present invention containing the specific combination of compounds, so that a person skilled in the art being aware of the previously cited prior art would NOT have any way to anticipate the inventive and unexpected combination of amount of the compounds, in particular, in their claimed concentrations ranges, in an obvious manner, for anti-aging properties, and for reducing the carbonylation of skin proteins, and for improving and restoring the skin barrier, hydration of the skin, ensuring a certain cohesion and a strengthening of the dermo-epidermal junction and the extracellular matrix, anti-inflammatory benefits, anti-oxidant and detoxification benefits, anti-microbial benefits and anti-pigmentation properties.

The following list of extracts of Ash bark can be used in the present invention:
Fraxinus excelsior, Fraxinus apelata, Fraxinus ararica, Fraxinus atrovirens, Fraxinus anomala, Fraxinus dipetala, Fraxinus quadrangulata, Fraxinus mandshurica, Fraxinus nigra, Fraxinus platypoda, Fraxinus americana, Fraxinus Berlandieriana, Fraxinus Caroliniana, Fraxinus Latifolia, Fraxinus Papillosa, Fraxinus Pennsylvanica, Fraxinus Profunda, Fraxinus Texensis, Fraxinus Uhdei, Fraxinus Velutina, Fraxinus Apertisquamifera, Fraxinus Bungeana, Fraxinus Floribunda, Fraxinus Griffithii, Fraxinus Lanuginoa, Fraxinus Malacophylla, Fraxinus Ornus, Fraxinus Paxiana, Fraxinus Raibocarpa, Fraxinus Sieboldina, Fraxinus Trifoliolata, Fraxinus Baroniana, Fraxinus Chinensis, Fraxinus Longicuspis, Fraxinus Micrantha, Fraxinus Dubia, Fraxinus Gooddingii, Fraxinus Greggii, Fraxinus Purpusii, Fraxinus Americana L., Fraxinus angustifolia, Fraxinus pennsylvanica Marshall.

The following list of extracts of walnut oil can be used in the present invention:
Juglans regia, Juglans amara, Juglans illinoinensis, Juglans ovata, Juglans alba, Juglans ailantifolia, Juglans fallax, Juglans x sinensis, Juglans australis, Juglans mandshurica, Juglans nigra, Juglans venezuelensis, Juglans fraxinifolia.

The origin of Ash bark (Fraxinus excelsior) and Walnut seed oil (Juglans regia) is from France.

### Detailed description of the invention:

### Definitions:

The present invention contains four essential ingredients, i.e. Ash bark (Fraxinus excelsior) and Walnut seed oil (Juglans regia) and Polyglyceryl-4 oleate and Tocopherol which must be present in the intermediary cosmetic composition and in the final cosmetic product composition of the present invention.

The wording "active ingredient" means an extract of Fraxinus Excelsior Ash bark.

The wording "non-active ingredients" means natural and/or chemical compounds in an indefinite number (preferably from 3 to 5030 ingredients) and which are different from the extract of Fraxinus Excelsior Ash bark (active ingredient). Walnut seed oil (Juglans regia) and Polyglyceryl-4 oleate and Tocopherol are non-active ingredients.

The wording "final cosmetic product" in the present invention means the final product as applied on the skin of the human being or animal in the form of a cream, or an emulsion, or an oil, or a serum, or a gel, or a spray, or a balm, or a lotion, or a stick, or a foam, or a shower gel, and containing the active ingredient and at least three non-active ingredients which are preferably in a number from 3 to 5030.

The wording "OLEAA" means the combination of the four essential ingredients i.e. Ash bark extract (Fraxinus excelsior) and Walnut seed oil (Juglans regia) and Polyglyceryl-4 oleate and tocopherol. An **extract** refers to a concentrated substance obtained from a natural source such as plants, flowers, fruits, algae, or even animal-derived ingredients, through various extraction processes. These extracts contain beneficial active compounds that provide skincare or haircare benefits.

**Table 1: Example of Ash bark extract (Fraxinus excelsior) and Walnut seed oil (Juglans regia) for cosmetic applications in an intermediary cosmetic composition**

| **Ingredients** | **Content in weight** % | | | | | | | |
|---|---|---|---|---|---|---|---|---|
| **Ash bark (Fraxinus excelsior) extract** | 30 | 29 | 28 | 27 | **26** | 25 | 24 | 20 |
| **Walnut seed oil (Juglans regia)** | 70 | 71 | 72 | 73 | **74** | 75 | 76 | 80 |

The content of Ash bark extract (Fraxinus excelsior) is preferably 1 wt%, or 2, 3, 4, 5, 6, 7, 8, 9, 10, 11, 12, 13, 14, 15, 16, 17, 18, 19, 20, 21, 22, 23, 24, 25, 26, 27, 28, 29, 30 wt%.

The content of Walnut seed oil (Juglans regia) is preferably 60 wt%, or 61, 62, 63, 64, 65, 66, 67, 68, 69, 70, 71, 72, 73, 74, 75, 76, 77, 78, 79, 80 wt%.

Any specific previously mentioned content of Fraxinus excelsior can be combined with any other specific previously mentioned content of Walnut seed oil to form a new composition (e.g. 20+80 = 100wt%) as well as to form any new range not disclosed in the claims.

**Table 2: Example of an extract of Ash bark extract (Fraxinus excelsior) and Walnut seed oil (Juglans regia) and Polyglyceryl-4 oleate for cosmetic applications in an intermediary cosmetic composition.**

| **Ingredients** | **Content in weight %** | | | | | | | | |
|---|---|---|---|---|---|---|---|---|---|
| **Ash bark (Fraxinus excelsior) extract** | 15 | 14 | 13 | 12 | **11** | 10 | 9 | 8 | 7 |
| **Walnut seed oil (Juglans regia)** | 60 | 62 | 65 | 70 | **71** | 72 | 75 | 77 | 80 |
| **Polyglyceryl-4 oleate** | 25 | 24 | 22 | 18 | **18** | 18 | 16 | 15 | 13 |

Ash bark extract (Fraxinus excelsior) and Walnut seed oil (Juglans regia) must be present in the ingredients of the cosmetic composition of the present invention. Polyglyceryl-4 oleate is used as a co-extractant, improving the content of molecules of interest in the cosmetic composition described later in the description. Polyglyceryl-4 oleate is added during the extraction of molecules of interest from ash bark into walnut seed oil.

The content of Ash bark extract (Fraxinus excelsior) is preferably 1 wt%, or 2, 3, 4, 5, 6, 7, 8, 9, 10, 11, 12, 13, 14, 15, 16, 17, 18, 19, 20, 21, 22, 23, 24, 25, 26, 27, 28, 29, 30 wt%.

The content of Walnut seed oil (Juglans regia) is preferably 60 wt%, or 61, 62, 63, 64, 65, 66, 67, 68, 69, 70, 71, 72, 73, 74, 75, 76, 77, 78, 79, 80 wt%.

The content of Polyglyceryl-4 oleate is preferably 5 wt%, or 6, 7, 8, 9, 10, 11, 12, 13, 14, 15, 16, 17, 18, 19, 20, 21, 22, 23, 24, 25, 26, 27, 28, 29, 30 wt%.

Any specific previously mentioned ingredient amount can be combined with any other specific ingredient amount previously mentioned to form a new composition amount (e.g. 15+70+15 = 100wt% or 7+80+13 = 100 wt%) as well as to form any new range not disclosed in the claims.

**Table 3: Example of the composition of OLEAA for cosmetic composition integration. OLEAA is composed of Ash bark extract (Fraxinus excelsior) and Walnut seed oil (Juglans regia) and Polyglyceryl-4 oleate and tocopherol in an intermediary cosmetic composition.**

| | **Ingredients** | **Content in weight %** | | | | | | | | |
|---|---|---|---|---|---|---|---|---|---|---|
| **OLEA A** | **Ash bark (Fraxinus excelsior) extract** | 11 | 11 | 11 | 11 | 11 | 11 | 11 | 11 | 11 |
| | **Walnut seed oil (Juglans regia)** | 71 | 71 | 71 | 71 | 71 | 71 | 71 | 71 | 71 |
| | **Polyglyceryl -4 oleate** | 17,90 | 17,88 | 17,84 | 17,80 | 17,78 | 17,76 | 17,74 | 17,72 | 17,70 |
| | **Tocopherol** | 0,10 | 0,12 | 0,16 | 0,20 | 0,22 | 0,24 | 0,26 | 0,28 | 0,30 |

Tocopherol acts as a stabilizing ingredient. Tocopherol is added after the extraction of molecules of interest from ash bark into walnut seed oil, assisted by the co-extractant polyglyceryl-4 oleate.

**In an intermediary cosmetic composition:**
- the content of Ash bark extract (Fraxinus excelsior) is preferably 1 wt%, or 2, 3, 4, 5, 6, 7, 8, 9, 10, 11, 12, 13, 14, 15, 16, 17, 18, 19, 20, 21, 22, 23, 24, 25, 26, 27, 28, 29, 30 wt%.
- the content of Walnut seed oil (Juglans regia) is preferably 60 wt%, or 61, 62, 63, 64, 65, 66, 67, 68, 69, 70, 71, 72, 73, 74, 75, 76, 77, 78, 79, 80 wt%.
- the content of Polyglyceryl-4 oleate is preferably 5 wt%, or 6, 7, 8, 9, 10, 11, 12, 13, 14, 15, 16, 17, 18, 19, 20, 21, 22, 23, 24, 25, 26, 27, 28, 29, 30 wt%.
- the content of Tocopherol is preferably: 0,10 wt%, or 0,11, 0,12, 0,13, 0,14, 0,15, 0,16, 0,17, 0,18, 0,19, 0,20, 0,21, 0,22, 0,23, 0,24, 0,25, 0,26, 0,27, 0,28, 0,29, 0,30, 0,35, 0,40, 0,45, 0,50, 0,55, 0,60, 0,65, 0,70, 0,75, 0,80, 0,85, 0,90, 1,0 wt%.

Any specific previously mentioned ingredient amount can be combined with any other specific ingredient amount previously mentioned to form a new composition amount as well as to form any new range not disclosed in the claims.

**In a final cosmetic product composition:**
- the content of Ash bark extract (Fraxinus excelsior) is preferably 0,09 wt%, or 0,10, 0,11, 0,12, 0,13, 0,14, 0,15, 0,16, 0,17, 0,18, 0,19, 0,20, 0,21, 0,22, 0,23, 0,24, or 0,25 wt% of the final cosmetic product composition.
- the content of Walnut seed oil (Juglans regia) is preferably 0,69 wt%, or 0,70, 0,71, 0,72, 0,73, 0,74, 0,75, 0,76, 0,77, 0,78, 0,79, 0,80, 0,81, 0,82, 0,83, 0,84, 0,85, 0,86, 0,87, 0,88, 0,89, 0,90, 0,91, 0,92, 0,93, 0,94, 0,95, 0,96, 0,97, 0,98, 0,99, 1,00, 1,01, 1,02, 1,03, 1,04, 1,05, 1,06, 1,07, 1,08, 1,09, 1,10, 1,11, 1,12, 1,13, 1,14, 1,15, 1,16, 1,17, 1,18, 1,19, 1,20, 1,21, 1,22, 1,23, 1,24, 1,25, 1,26, 1,27, 1,28, 1,29, 1,30, 1,31, 1,32, 1,33, 1,34, 1,35, 1,36, 1,37, 1,38, 1,39, 1,40, 1,41, 1,42, 1,43, 1,44, 1,45, 1,46, 1,47, 1,48, 1,49, or 1,50 wt% of the final cosmetic product composition.
- the content of Polyglyceryl-4 oleate is preferably 0,16 wt%, or 0,17, 0,18, 0,19, or 0,20 wt% of the final cosmetic product composition.
- the content of Tocopherol is preferably: 0,001 wt%, or 0,002, 0,003, 0,004, 0,005, 0,006, 0,007, 0,008, 0,009, 0,010, 0,011, 0,012, 0,013, 0,014, 0,015, 0,016, 0,017, 0,018, 0,019, 0,020, 0,021, 0,022, 0,023, 0,024, 0,025, 0,026, 0,027, 0,028, 0,029, 0,030, 0,031, 0,032, 0,033, 0,034, 0,035, 0,036, 0,037, 0,038, 0,039, 0,040, 0,041, 0,042, 0,043, 0,044, 0,045, 0,046, 0,047, 0,048, 0,049, 0,050, 0,051, 0,052, 0,053, 0,054, 0,055, 0,056, 0,057, 0,058, 0,059, 0,060, 0,061, 0,062, 0,063, 0,064, 0,065, 0,066, 0,067, 0,068, 0,069, 0,070, 0,071, 0,072, 0,073, 0,074, 0,075, 0,076, 0,077, 0,078, 0,079, 0,080, 0,081, 0,082, 0,083, 0,084, 0,085, 0,086, 0,087, 0,088, 0,089, 0,090, 0,091, 0,092, 0,093, 0,094, 0,095, 0,096, 0,097, 0,098, 0,099, 0,100, 0,150, 0,200, 0,250, 0,300, 0,350, 0,400, 0,450, 0,500, 0,550, 0,600, 0,650, 0,700, 0,710, 0,720, or 0,730 wt% of the final cosmetic product composition.
- the rest of the final cosmetic product composition being one or more other ingredients than Ash bark extract and Walnut seed oil and Polyglyceryl-4 oleate and Tocopherol, independently of their amount but the total amount of the final cosmetic product composition should be 100 wt%. It could also be an acceptable cosmetical carrier.

Any specific previously mentioned ingredient amount can be combined with any other specific ingredient amount previously mentioned to form a new composition amount as well as to form any new range not disclosed in the claims.

The leaves and bark of the ash tree were analyzed in parallel to determine whether one of the two parts of the tree would be more suitable for the development of this active ingredient:
- Appearance, color, odor,
- Water content,
- Spectrophotometric determination of total phenols (Folin assay),
- Quantification of a specific biomarker by HPLC.

The parts of the ash tree were analyzed to determine which part had the highest polyphenol content. The list of plant parts analyzed using the Total Polyphenol Content Assay (Folin-Ciocalteu) method is found at Table 4A:

**Table 4A:**

| Batch number | Photograph on receipt | Appearance, Color, Odor | | Water Content (%) | Total Polyphenols in eq. Oleuropein (mg/kg of product) ± 20% |
|---|---|---|---|---|---|
| FFREb130421-1 | A in Figure 39 | Leaf pieces | | 10.1 | 63400 (6.3%) |
| | | | Green to brown for the leaflets | | |
| | | | Green to brown-beige for the petioles | | |
| | | | Vegetal, herbaceous | | |
| FFEb190521-1 | B in Figure 39 | Leaf pieces | | 7.9 | 82460 (8.2%) |
| | | | Green to brown for the leaflets | | |
| | | | Green to brown for the petioles | | |
| | | | Vegetal, herbaceous | | |
| EFREb040521-1 | C in Figure 39 | External face gray/brown | | 8.1 | 81100 (8.1%) |
| | | | Internal face beige | | |
| | | | Presence of gray/yellow lichen in places | | |
| EFRE280122-1 | D in Figure 39 | Bark pieces | | 9.7 | 125300 (12.5%) |
| | | Beige/off-white for the mass, shades of black and brown for the larger pieces | | | |
| | | Mild woody | | | |

Ash bark extract (Fraxinus Excelsior) from 2 different batches have been analyzed by High Performance Liquid Chromatography (HPLC). The following components molecules of interest have been detected and described below:
Fraxin was identified in the two batches of the Fraxinus excelsior bark.

Fraxin is a common group of coumarin molecules. In the literature of pharmacological and medical domain, Fraxin was described to have anti-inflammatory, antioxidant and anti-microbial properties. Fraxin isn't well known in cosmetic domain and not described for anti-aging properties. In hence, the presence of Fraxin in the cosmetic composition was unknown and could predict good properties for the skin and more preferentially for anti-aging properties.

**Table 4B: Characteristics of OLEAA:**

| | |
|---|---|
| **Aspect at 25°C** | Homogenous, fluid, clear |
| **Color** | Dark brown green |
| **Odor** | Mean intensity, herbaceous, fat |
| **Peroxide index (meqO₂/kg)** | 10 |
| **Total content in polyphenol (mg/kg eq. Oleuropein) +/- 20% (n=1)** | 6440 +/- 1290 |

### Conclusion:

The preferred cosmetic composition with OLEAA integration has a high content in polyphenol and a rich phytochemical profile which confirms the doses results of the total polyphenols.

The peroxide index is an indicator to evaluate the rancidity of a fatty substance. Oils below 10 meqO₂/kg are considered fresh. The preferred cosmetic composition is therefore not rancid and acceptable for a cosmetic application.

The organoleptic characteristics are acceptable for a cosmetic application according to internal criteria.

**Table 5: choice of the co-extractant for OLEAA:**

| **Test** | **Test 2** | **Test 3** | **Test 4** | **Test 5** | **Test 6** |
|---|---|---|---|---|---|
| **Co extractant** | - | - | Polyglyceryl-3-diisosterate (20%) | **Polyglyceryl-4-oleate (20%)** | Polyglyceryl -6-distearate (20%) |
| **Aspect (25°C)** | homogenous, fluid, clear | homogenous, fluid, clear | homogenous, fluid, clear | homogenous, fluid, clear | |
| **Color** | Light yellow | Light yellow | Light yellow | Light yellow | |
| **Odor** | Woody nut | Woody nut | Woody nut | Woody nut | |
| **Peroxide index (meqO₂/kg)** | 5 +/- 2,0 | 5 +/- 2,1 | 3 +/- 1,4 | 3 +/- 1,2 | |
| **Peroxide index after 2 weeks at 40°C (meqO₂/kg)** | 10 +/- 4,0 | 14 +/- 4,0 | 3 +/- 1,2 | 3 +/- 1,1 | |
| **Total content in total polyphenol s (mg/kg eq. Oleuropein) +/- 20% (n=2)** | 310 | 230 | 2970 | **5900** | < 2970 because partly soluble in oil |

### Conclusion for Table 5:

Test 5 (Polyglyceryl-4-oleate) has been selected in the present invention because it has an acceptable peroxide index and the highest polyphenols content (5900).

The closest prior art contains Polyglyceryl-6-distearate and it can be concluded that the polyphenols content (< 2970 ) is much less than the polyphenols content (5900) for Polyglyceryl-4-oleate.

Polyglyceryl-4 oleate (HLB=4) which is readily oil-soluble and able to disperse hydrophilic compounds, extracts twice the amount of polyphenols compared to Polyglyceryl-3 diisostearate (HLB=7.5).

The addition of a co-extractant, whether Polyglyceryl-3 diisostearate (PG3DS) or Polyglyceryl-4 oleate (PGMO), significantly improves the extraction performance compared to a trial without a co-extractant. The use of the PGMO walnut oil solvent yields the best results. This confirms that surfactants used as co-extractants help migrating polyphenols toward the oil solvent. The total content in total polyphenols seems to be influenced by the HLB of the surfactant. The trials are non-oxidized at the end of production. Trials containing 20% co-extractant show the lowest peroxide index values.

Increasing the proportion of the co-extractant in the solvent enhances the oxidative stability of the trials, regardless of the co-extractant used.

A synergistic effect has been demonstrated between microwave treatment and ultrasound treatment, allowing for an improved extraction yield in a shorter time and with a reduced amount of fat, which enhances the final concentration of extracted active molecules. The OLEOS process according to the patented invention makes it possible to obtain an oily extract concentrated in active ingredients which can be in the form of an oily solution, micro dispersion oily, oily micro suspension or oily microemulsion, whatever form stable over time.

### Choice of the stabilizer toward rancidity: tocopherol or rosemary extract

Vegetable oils are subject to rancidity, which alters their organoleptic properties, impacts their shelf life and their possible use for cosmetic applications. It was therefore chosen to add a stabilizer to the preferred cosmetic composition. Several stability tests were performed on a tocopherol and a supercritical rosemary extract CO₂. These stabilizers are commonly used in cosmetic compositions. Stability results are similar between tocopherol and rosemary extract. Tocopherol was chosen to facilitate the INCI list of the finished product.

Qualitative and quantitative analysis of the OLEAA integrated in cosmetic composition:
OLEAA has been analyzed by Gas Chromatography associated with Mass spectrometry (GS-MS) and High-Performance Liquid Chromatography (HPLC). These analyzes are used to identify organic compounds in OLEAA.

The following molecules of interest have been detected:

**Table 6: Molecules of interest contained in OLEAA**

| **Name** | **Formula** | **N°CAS** | **Quantification** |
|---|---|---|---|
| Fraxin | C₁₆H₁₈O₁₀ | 524-30-1 | < 100 ppm |
| Fraxinol | C₁₁H₁₀O₅ | 486-28-2 | / |
| Isofraxidin | C₁₁H₁₀O₅ | 486-21-5 | / |
| Flavaprin | C₂₆H₃₀O₁₀ | 53846-49-4 | / |
| Icaritin 3-rhamnoside | C₂₇H₃₂O₁₁ | 108195-76-2 | / |
| Machaerol B | C₁₉H₂₂O₇ | / | / |
| Phloretin 2"-O-(6-O-acetylglucosid) | C₂₃H₂₆O₁₁ | **60-81-1** | / |

### Conclusion of Table 6:

OLEAA contains the following 6 molecules of interest: Fraxin, Flavaprin, Icaritin 3-rhamnoside, Machaerol B, Phloretin 2"-O-(6-O-acetylglucosid).

Flavonoids are a family of polyphenols, molecules of interest for cosmetic application.

Fraxin is identified as molecule of interest in the Fraxinus Excelsior bark, these studies have demonstrated the presence of Fraxin in OLEAA composition. Like described in literature, Fraxin is an interesting molecule for anti-inflammatory, antioxidant and anti-microbial properties.

Fraxinol and Isofraxin were identified and come from derivatives compounds of Fraxin. Their properties are similar to Fraxin.

Machaerol B is a phytocannabinoïd. It could have an impact on endocannabinoid biologic pathways.

Phloretin 2"-O-(6-O-acetylglucosid) is known to have properties on skin aging by reducing inflammation markers, skin pigmentation with an action on tyrosinase activity and acne pone skin with a diminution of *C.Acnes.*

The analytical analysis proves the preservation of the Fraxin present in OLEAA. Furthermore, the other components could predict a promising efficacy on skincare applications with anti-aging, anti-inflammatory, antioxidant, brightening, anti-microbial properties.

### Manufacturing process:

The process of extraction is related to a patented process hold by an active ingredient supplier, HALLSTAR (FR2943684).

One possible manufacturing process of the claimed composition is described as follows:
Oily extract of ash bark, in particular of the species Fraxinus excelsior, obtained by extraction by means of a natural fat, and advantageously in addition of a surfactant as a co-extractant, characterized in that it is obtained by means of a process extraction including the following steps:
(a) mixing and impregnating ash bark powder with the natural fat (Walnut seed oil) and co-extractant (Polyglycery-4 oleate) at a temperature higher than the melting point of the mixture and in an atmosphere without or essentially oxygen-free,
(b) the micro-dispersion of ash bark powder into the liquid mixture and under atmosphere without or essentially without oxygen, in particular by ultrasounds,
(c) heating the mixture thus obtained at a temperature of between 60°C and 150°C, in an atmosphere without or essentially without oxygen, in particular by using microwaves, Step (c) can be done before, during or after Step (b).
(d) the optional addition of a stabilizer.

The oil/co-extractant ratio is: Oil 95-75% / 5-25% co-extractant.

The fatty substance which is used as extraction solvent is a fatty compound, chosen from fatty substances of the glyceridic type; fatty substances of the non-glyceridic type; or a mixture and/or in that the first material of natural origin is chosen from terrestrial plants, and from aerial vegetative parts: leaves, stalks, flowers, whole plants, or even roots, tubers, grains, fruits, cattle cake, flours and any vegetable co-product; microscopic and macroscopic algae; mushrooms; lichens; bee-keeping products; minerals, rocks, sands or any mixture of these compounds and/or in that the natural non-volatile compounds which are extracted are any types of non-volatile compounds chosen from lipo-soluble, lipo-extractable or lipo-dispersible compounds.

The natural non-volatile extracted compounds are nonpolar, polar or amphiphilic molecules chosen from phenolic compounds, of the polyphenol, flavonoid, phenolic acid, catechin, diterpene, flavone, monophenol, glycoside flavonol type; vitamins of the free A, E, C vitamin type or esters; tannins; waxes; fatty acids; essential oils; organic acids; hydrophobic proteins; pigments; non-saponifiable compounds; polar lipids; enzymes and coenzymes; oligo-elements; minerals; organic salts or a mixture of any of these compounds.

Characteristics of the ingredients of OLEAA:
- The active ingredient is ash bark which is obtained through a patented process by the supplier called oleo-eco-extraction.
- The solvent used is walnut oil for fatty substance.
- The natural surfactant used is Polyglyceryl-4 oleate.
- All ingredients are sourced from France and certified organic.
- The bark is upcycled: it comes from wood cultivation for the manufacture of tool handles.
- Benefits of oleo-eco-extraction:
   - obtaining an oil extract rich in beneficial molecules,
   - no chemical additives,
   - no heating,
   - low energy consumption,
   - green chemistry process.

### Different final cosmetic product compositions:

Some examples of final cosmetic compositions of the present invention are described hereunder. OLEAA is integrated in the final cosmetic product formulations. The quantity of each compound is described in the tables below.

**Table 7: thick final cosmetic product composition (thick cream):**

| ***Phase*** | ***INCI EU*** | ***Example 1:** wt % matter* | ***Example 2:** wt % matter* | ***Example 3A:** wt % matter* | ***Example 3B:** wt % matter* |
|---|---|---|---|---|---|
| A | AQUA (WATER) | 77,555 | 77,555 | 77,555 | 77,555 |
| B | ACRYLATES/C10-30 ALKYL ACRYLATE CROSSPOLYMER | 0,400 | 0,400 | 0,400 | 0,400 |
| C | XANTHAN GUM | 0,150 | 0,150 | 0,150 | 0,150 |
| C | GLYCERIN | 3,000 | 3,000 | 3,000 | 3,000 |
| D | PHENOXYETHANOL | 0,400 | 0,400 | 0,400 | 0,400 |
| D | SODIUM GLUCONATE | 0,200 | 0,200 | 0,200 | 0,200 |
| D | CAPRYLOYL GLYCINE | 0,200 | 0,200 | 0,200 | 0,200 |
| D | CITRIC ACID | 0,120 | 0,120 | 0,120 | 0,120 |
| E | AQUA (WATER) | 0,900 | 0,900 | 0,900 | 0,900 |
| E | SODIUM HYDROXIDE | 0,225 | 0,225 | 0,225 | 0,225 |
| F | ARACHIDYL ALCOHOL BEHENYL ALCOHOL ARACHIDYL GLUCOSIDE | 3,000 | 3,000 | 3,000 | 3,000 |
| F | SODIUM STEAROYL GLUTAMATE | 0,350 | 0,350 | 0,350 | 0,350 |
| F | CETEARYL ALCOHOL | 1,500 | 1,500 | 1,500 | 1,500 |
| F | SQUALANE | 6,000 | 5,000 | 4,000 | 4,000 |
| F | DIMETHICONE | 2,000 | 2,000 | 2,000 | 2,000 |
| F | DEHYDROACETIC ACID | 0,200 | 0,200 | 0,200 | 0,200 |
| F | TOCOPHERYL ACETATE | 0,500 | 0,500 | 0,500 | 0,500 |
| F | MACADAMIA INTEGRIFOLIA SEED OIL | 2,500 | 2,500 | 2,500 | 2,500 |
| G | SILICA | 0,500 | 0,500 | 0,500 | 0,500 |
| G | ETHYLHEXYLGLYCERIN TOCOPHEROL | 0,300 | 0,300 | 0,300 | 0,300 |
| H | OLEAA | **0,000** | **1,000** | **2,000** | **2,000** |
| | Total | **100** | **100** | **100** | **100** |

Phase B is weighed and added to Phase A under agitation (Rayneri equipped with a tooth propeller) at 400 rpm.

Mixture phase A+ phase B is heated to 80°C.

Phase C gelling agent is premixed in glycerin and added to phase A+ phase B at 700 rpm for 10 minutes.

Phase D ingredients are added to the mixture at 400 rpm for 10 minutes.

Phase E is added to the mixture to adjust the pH at 1800 rpm, for 10 minutes.

Phase F is heated to 80°C in a water bath.

Phase F is added to the mixture at 2500 rpm for 15 minutes.

The mixture is cooled to 50°C under stirring at 1500 rpm.

Phase G is added to the mixture at 1500 rpm for 10 minutes.

Phase H is added to the mixture at 700 rpm for 10 minutes.

Phase H contains:
Example 1: 0 wt% of an extract of Fraxinus Excelsior Ash bark + 0 wt% of Juglans Regia walnut seed oil + 0 wt% of polyglyceryl-4 oleate + 0 wt% of tocopherol.
Example 2: 0,11 wt% of an extract of Fraxinus Excelsior Ash bark + 0,71 wt% of Juglans Regia walnut seed oil + 0,177 wt% of polyglyceryl-4 oleate + 0,003 wt% of tocopherol.
Example 3A: 0,22 wt% of an extract of Fraxinus Excelsior Ash bark + 1,42 wt% of Juglans Regia walnut seed oil + 0,354 wt% of polyglyceryl-4 oleate + 0,006 wt% of tocopherol.
Example 3B: 0,22 wt% of an extract of Fraxinus Excelsior Ash bark + 1,42 wt% of Juglans Regia walnut seed oil + 0,360 wt% of polyglyceryl-4 oleate + 0 wt% of tocopherol.

The mixture is cooled to 30°C under stirring at 600 rpm, the final pH is between 5.5 and 6.0.

Viscosities are measured on a Brookfield viscometer at a speed of 10 rpm, mobile rv-6. On the same day the viscosity value is :
Example 1: 37100 cP.s for a torque of 37.1%, the next day the viscosity value is 38600 cP.s for a torque of 38.6%.
Example 2: 30900 cP.s for a torque of 30.9%, the next day the viscosity value is 35000 cP.s for a torque of 35%.
Examples 3A and 3B: 28800 cP.s for a torque of 28.8%, the next day the viscosity value is 34400 cP.s for a torque of 34.4%.

The appearance is that of a homogeneous thick emulsion.

Stability tests were carried at 4°C, ambient temperature, 40°C/75% humidity, 50°C, light for 3 months: the cream darkens very slightly at 40°C and darkens at 50°C. Stability is acceptable according to internal criteria for appearance, colour, odour, viscosity and pH. The centrifugation test (3 cycles of 10 min at 4000 rpm) at 24 H is acceptable because there is no phase separation.

Example 1: The pH of the final cosmetic product has a value of 5,66 the same day, the next day, the value of it is 5,63.

Example 2: The pH of the final cosmetic product has a value of 5,67 the same day, the next day, the value of it is 5,62.

Examples 3A and 3B: The pH of the final cosmetic product has a value of 5,71 the same day, the next day, the value of it is 5,64.

**Table 8: light final cosmetic product (light cream):**

| ***Phase*** | ***INCI EU*** | ***Example 4:** wt % Matter* | ***Example 5:** wt % Matter* | ***Example 6:** wt % Matter* | ***Example 6:** wt % Matter* |
|---|---|---|---|---|---|
| A | AQUA (Water) | 66,70 | 66,70 | 66,70 | 66,70 |
| B | GLYCERIN | 3,00 | 3,00 | 3,00 | 3,00 |
| B | SPHINGOMONAS FERMENT EXTRACT | 0,10 | 0,10 | 0,10 | 0,10 |
| B | CAESALPINIA SPINOSA GUM | 0,40 | 0,40 | 0,40 | 0,40 |
| C | GLYCERYL STEARATE CITRATE | 4,00 | 4,00 | 4,00 | 4,00 |
| C | COCO-CAPRYLATE/CAPRATE | 25,00 | 24,00 | 23,00 | 23,00 |
| D | PHENETHYL ALCOHOL | 0,30 | 0,30 | 0,30 | 0,30 |
| D | ETHYLHEXYLGLYCERIN | 0,50 | 0,50 | 0,50 | 0,50 |
| E | OLEAA | **0,000** | **1,000** | **2,000** | **2,000** |
| | Total | **100** | **100** | **100** | **100** |

Phase A is weighed and agitated with a rotor-stator at 150 rpm and heated to 80°C.

Phase B gelling agents are premixed in glycerin and added to phase A at 500 rpm for 10 minutes.

Phase C is heated to 80°C in a water bath.

Phase C ingredients are added to the mixture at 1500 rpm, for 10 minutes.

The mixture is cooled to 30°C under stirring (tooth propeller) at 200 rpm.

Phase D is added to the mixture at 200 rpm for 10 minutes, the final pH is between 5.0 and 5.5 (if no phase E).

Phase E is added to the mixture at 200 rpm for 10 minutes, the final pH is between 5.0 and 5.5.

Phase E contains:
Example 4: 0 wt% of an extract of Fraxinus Excelsior Ash bark + 0 wt% of Juglans Regia walnut seed oil + 0 wt% of polyglyceryl-4 oleate + 0 wt% of tocopherol.
Example 5: 0,11 wt% of an extract of Fraxinus Excelsior Ash bark + 0,71 wt% of Juglans Regia walnut seed oil + 0,177 wt% of polyglyceryl-4 oleate + 0,003 wt% of tocopherol.
Example 6A: 0,22 wt% of an extract of Fraxinus Excelsior Ash bark + 1,42 wt% of Juglans Regia walnut seed oil + 0,354 wt% of polyglyceryl-4 oleate + 0,006 wt% of tocopherol.
Example 6B: 0,22 wt% of an extract of Fraxinus Excelsior Ash bark + 1,42 wt% of Juglans Regia walnut seed oil + 0,360 wt% of polyglyceryl-4 oleate + 0 wt% of tocopherol.

Viscosities are measured on a Brookfield viscometer at a speed of 20 rpm, mobile rv-5.

On the same day the viscosity value is :
Example 4: 4200 cP. s for a torque of 21%, the next day the viscosity value is 4540 cP. s for a torque of 22,7%.
Example 5: 4360 cP. s for a torque of 21,8%, the next day the viscosity value is 4560 cP. s for a torque of 22,8%.
Examples 6A and 6B: 4300 cP. s for a torque of 21,5%, the next day the viscosity value is 4700 cP. s for a torque of 23,5%.

The appearance is that of a homogeneous fluid emulsion.

Stability tests were carried at 4°C, Room temperature, 40°C/75% humidity, 50°C, Light for 3 months: the cream darkens at 50°C and the pH decreases slightly over time at room temperature. Stability is acceptable based on internal criteria for appearance, colour, odour, viscosity and pH. The centrifugation test (3 cycles of 10 min at 4000 rpm) at 24 H is acceptable because there is no phase separation.

Example 4: the pH of the cream has a value of 5,47 the same day, the next day, the value of it is 5,38.

Example 5: the pH of the cream has a value of 5,45 the same day, the next day, the value of it is 5,38.

Examples 6A and 6B: the pH of the cream has a value of 5,46 the same day, the next day, the value of it is 5,42.

**Table 9: final cosmetic product composition (plumping cream):**

| ***Phase*** | ***INCI EU*** | ***Example 7:** wt % Matter* | ***Example 8:** wt % Matter* | ***Example 9A:** wt % Matter* | ***Example 9B:** wt % Matter* |
|---|---|---|---|---|---|
| A | AQUA (Water) | 68,42 | 68,42 | 68,42 | 68,42 |
| A | p-ANISIC ACID | 0,20 | 0,20 | 0,20 | 0,20 |
| A | SODIUM HYDROXIDE | 0,10 | 0,10 | 0,10 | 0,10 |
| A | PENTYLENE GLYCOL | 2,00 | 2,00 | 2,00 | 2,00 |
| A | SODIUM GLUCONATE | 0,20 | 0,20 | 0,20 | 0,20 |
| B | LEVULINIC ACID | 0,70 | 0,70 | 0,70 | 0,70 |
| | GLYCERIN | | | | |
| | SODIUM LEVULINATE | | | | |
| | AQUA | | | | |
| C | GLYCERIN | 4,18 | 4,18 | 4,18 | 4,18 |
| C | XANTHAN GUM | 0,40 | 0,40 | 0,40 | 0,40 |
| D | DICAPRYLYL ETHER | 4,00 | 4,00 | 4,00 | 4,00 |
| | TOCOPHEROL | | | | |
| D | CAPRYLIC/CAPRIC | 4,50 | 3,50 | 2,50 | 2,50 |
| | TRIGLYCERIDE | | | | |
| D | LAURYL LAURATE | 1,00 | 1,00 | 1,00 | 1,00 |
| D | GLYCERYL STEARATE | 3,00 | 3,00 | 3,00 | 3,00 |
| | CITRATE | | | | |
| D | CETEARYLALCOHOL | 1,00 | 1,00 | 1,00 | 1,00 |
| D | BEHENYL ALCOHOL | 3,00 | 3,00 | 3,00 | 3,00 |
| D | SODIUM STEAROYL GLUTAMATE | 0,50 | 0,50 | 0,50 | 0,50 |
| D | TOCOPHEROL | 0,20 | 0,20 | 0,20 | 0,20 |
| D | BUTYROSPERMUM PARKII BUTTER | 3,00 | 3,00 | 3,00 | 3,00 |
| D | GLYCERYL CAPRYLATE | 0,30 | 0,30 | 0,30 | 0,30 |
| D | PRUNUS AMYGDALUS DULCIS OIL | 3,00 | 3,00 | 3,00 | 3,00 |
| E | ETHYLHEXYLGLYCERIN | 0,30 | 0,30 | 0,30 | 0,30 |
| F | OLEAA | **0,00** | **1,00** | **2,00** | **2,00** |
| | Total | **100** | **100** | **100** | **100** |

Phase A ingredients are weighed and stirred (rotor stator) at 200 rpm and heated to 80°C.

Phase B is added to phase A under stirring at 200 rpm.

Phase C gelling agent is premixed in glycerin and then added to A+B at 1000 rpm for 10 minutes.

Phase D is heated to 80°C in a water bath.

Phase D ingredients are added to the mixture at 2000 rpm, for 15 minutes.

The mixture is cooled to 30°C under stirring (tooth propeller) at 600 rpm.

Phase E is added to the mixture at 500 rpm for 10 minutes.

Phase F is added to the mixture at 500 rpm for 10 minutes.

The final pH is between 5.0 and 5.5.

Phase F contains:
Example 7: 0 wt% of an extract of Fraxinus Excelsior Ash bark + 0 wt% of Juglans Regia walnut seed oil + 0 wt% of polyglyceryl-4 oleate + 0 wt% of tocopherol.
Example 8: 0,11 wt% of an extract of Fraxinus Excelsior Ash bark + 0,71 wt% of Juglans Regia walnut seed oil + 0,177 wt% of polyglyceryl-4 oleate + 0,003 wt% of tocopherol.
Example 9A: 0,22 wt% of an extract of Fraxinus Excelsior Ash bark + 1,42 wt% of Juglans Regia walnut seed oil + 0,354 wt% of polyglyceryl-4 oleate + 0,006 wt% of tocopherol.
Example 9B: 0,22 wt% of an extract of Fraxinus Excelsior Ash bark + 1,42 wt% of Juglans Regia walnut seed oil + 0,360 wt% of polyglyceryl-4 oleate + 0 wt% of tocopherol.

Viscosities are measured on a Brookfield viscometer with a speed of 5 rpm, mobile rv-6. The same day the viscosity value is
Example 7: 16800 cP. s for a torque of 8,4%, the next day the viscosity value is 17200 cP. s for a torque of 8,6%.
Example 8: 19200 cP. s for a torque of 9,6%, the next day the viscosity value is 20800 cP. s for a torque of 10,4%.
Examples 9A and 9B: 17600 cP. s for a torque of 8,8%, the next day the viscosity value is 16800 cP. s for a torque of 8,4%.

The appearance is that of a homogeneous thick emulsion.

Stability tests were carried at 4°C, Ambient temperature, 40°C/75% humidity, 50°C, Light for 3 months: stability is acceptable according to internal criteria on appearance, colour, odour, viscosity and pH. The centrifugation test (3 cycles of 10 min at 4000 rpm) at 24 H is non-compliant because there is a phase separation.

Example 7: The pH of the final cosmetic product has a value of 5,48 the same day, the next day, the value of it is 5.48.

Example 8: The pH of the final cosmetic product has a value of 5,47 the same day, the next day, the value of it is 5,46.

Examples 9A and 9B: The pH of the final cosmetic product has a value of 5,47 the same day, the next day, the value of it is 5,46.

**Table 10: oil:**

| ***Phase*** | ***INCI EU*** | ***Example 10:** wt % Matter* | ***Example 11:** wt % Matter* | ***Example 12A:** wt % Matter* | ***Example 12B:** wt % Matter* |
|---|---|---|---|---|---|
| A | CAPRYLIC/CAPRIC TRIGLYCERIDE | 33,10 | 32,10 | 31,10 | 31,10 |
| A | COCO-CAPRYLATE/CAPRATE | 33,10 | 33,10 | 33,10 | 33,10 |
| A | PRUNUS AMYGDALUS DULCIS OIL | 33,10 | 33,10 | 33,10 | 33,10 |
| A | TOCOPHEROL HELIANTHUS ANNUUS SEED OIL | 0,70 | 0,70 | 0,70 | 0,70 |
| B | OLEAA | **0,00** | **1,00** | **2,00** | **2,00** |
| | Total | **100** | **100** | **100** | **100** |

Phase A ingredients are stirred (tooth propeller) at 300 rpm for 10 minutes.

Phase B is added to Phase A at 300 rpm for 10 minutes.

The appearance is that of a homogeneous oil.

Stability tests were carried out at 4°C, Ambient temperature, 40°C/75% humidity, 50°C, Light for 3 months: a slight and acceptable sedimentation appears at all conditions, stability is acceptable according to internal criteria on appearance, color, smell.

Phase B contains:
Example 10: 0 wt% of an extract of Fraxinus Excelsior Ash bark + 0 wt% of Juglans Regia walnut seed oil + 0 wt% of polyglyceryl-4 oleate + 0 wt% of tocopherol.
Example 11: 0,11 wt% of an extract of Fraxinus Excelsior Ash bark + 0,71 wt% of Juglans Regia walnut seed oil + 0,177 wt% of polyglyceryl-4 oleate + 0,003 wt% of tocopherol.
Example 12A : 0,22 wt% of an extract of Fraxinus Excelsior Ash bark + 1,42 wt% of Juglans Regia walnut seed oil + 0,354 wt% of polyglyceryl-4 oleate + 0,006 wt% of tocopherol.
Example 12B : 0,22 wt% of an extract of Fraxinus Excelsior Ash bark + 1,42 wt% of Juglans Regia walnut seed oil + 0,360 wt% of polyglyceryl-4 oleate + 0 wt% of tocopherol.

**Table 11: final cosmetic product composition (fluid serum):**

| ***Phase*** | ***INCI EU*** | ***Example 13:** wt% Matter* | ***Example 14:** wt% Matter* | ***Example 15A:** wt% Matter* | ***Example 15B:** wt% Matter* |
|---|---|---|---|---|---|
| A | AQUA (water) | 83,10 | 83,10 | 83,10 | 83,10 |
| B | SODIUM GLUCONATE | 0,20 | 0,20 | 0,20 | 0,20 |
| B | PENTYLENE GLYCOL | 2,00 | 2,00 | 2,00 | 2,00 |
| C | GLYCERIN | 4,00 | 4,00 | 4,00 | 4,00 |
| C | XANTHAN GUM | 0,40 | 0,40 | 0,40 | 0,40 |
| D | MICROCRYSTALLINE CELLULOSE CELLULOSE GUM | 1,50 | 1,50 | 1,50 | 1,50 |
| E | COCO-CAPRYLATE/CAPRATE TOCOPHEROL | **6,00** | **5,00** | **4,00** | **4,00** |
| E | GLYCERYL STEARATE CITRATE | 2,00 | 2,00 | 2,00 | 2,00 |
| F | CAPRYLYL GLYCOL 1,2-HEXANEDIOL TROPOLONE | 0,50 | 0,50 | 0,50 | 0,50 |
| F | ETHYLHEXYLGLYCERIN | 0,30 | 0,30 | 0,30 | 0,30 |
| G | OLEAA | **0,00** | **1,00** | **2,00** | **2,00** |
| | Total | **100** | **100** | **100** | **100** |

Phase A is weighed and shaken (rotor stator) at 150 rpm and heated to 80°C.

Phase B ingredients are added in A at 150 rpm, for 10 minutes.

Phase C gelling agent is premixed in glycerin and added to A+B at 900 rpm for 10 minutes. Phase D is added in A+B+C at 900 rpm, for 10 minutes.

Phase E is heated to 80°C in a water bath.

Phase E is added to A+B+C+D at 1500 rpm for 10 minutes.

The mixture is cooled to 30°C under stirring (tooth propeller) at 150 rpm.

Phase F is added to the mixture at 700 rpm for 10 minutes, the final pH is between 5,0 and 5,5.

Phase G is added to the mixture at 25°C 700 rpm for 10 minutes, the final pH is between 5,0 and 5,5.

Phase G contains:
Example 13: 0 wt% of an extract of Fraxinus Excelsior Ash bark + 0 wt% of Juglans Regia walnut seed oil + 0 wt% of polyglyceryl-4 oleate + 0 wt% of tocopherol.
Example 14: 0,11 wt% of an extract of Fraxinus Excelsior Ash bark + 0,71 wt% of Juglans Regia walnut seed oil + 0,177 wt% of polyglyceryl-4 oleate + 0,003 wt% of tocopherol.
Example 15A: 0,22 wt% of an extract of Fraxinus Excelsior Ash bark + 1,42 wt% of Juglans Regia walnut seed oil + 0,354 wt% of polyglyceryl-4 oleate + 0,006 wt% of tocopherol.
Example 15B: 0,22 wt% of an extract of Fraxinus Excelsior Ash bark + 1,42 wt% of Juglans Regia walnut seed oil + 0,360 wt% of polyglyceryl-4 oleate + 0 wt% of tocopherol.

Viscosities are measured on a Brookfield viscometer with a speed of 50 rpm, mobile rv-3. The same day the viscosity value is
Example 13: 676 cP. s for a torque of 33,8%, the next day the viscosity value is 890 cP. s for a torque of 44.5%.
Example 14: 680 cP. s for a torque of 34%, the next day the viscosity value is 890 cP. s for a torque of 44.5%.
Examples 15A and 15B: 676 cP. s for a torque of 33.8%, the next day the viscosity value is 890 cP. s for a torque of 44.5%.

The appearance is that of a homogeneous fluid emulsion.

Stability tests were carried out at 4°C, Ambient temperature, 40°C/75% humidity, 50°C, Light for 3 months: the formula darkens very slightly at 40°C and 50°C, the pH decreases slightly, and the viscosity increases slightly over time. Stability is acceptable based on internal criteria for appearance, colour, odour, viscosity and pH. Centrifugation test (3 cycles of 10 min at 4000 rpm) at 24 H is acceptable because there is no phase separation.

Example 13: The pH of the cream has a value of 5,50 the same day, the next day, the value of it is 5,49.

Example 14: The pH of the cream has a value of 5,50 the same day, the next day, the value of it is 5,49.

Examples 15A and 15B: The pH of the cream has a value of 5,49 the same day, the next day, the value of it is 5,48.

### In vitro efficacy tests on the expression of genes: comparative tests between a placebo and the cosmetic composition:

**Table 12: composition of the placebo: composition MD.01**

| **Phase** | **name** | **INCI** | **wt %** | **Quantity** | **unit** |
|---|---|---|---|---|---|
| A | Marcol B2 | Paraffinum Liquidum | 100 | 5 | g |

**Table 13: cosmetic composition diluted in liquid paraffin: composition MD.02**

| **Phase** | **name** | **INCI** | **wt %** | **Quantity** | **unit** |
|---|---|---|---|---|---|
| A | Marcol B2 | Paraffinum Liquidum | 99 | 4,95 | g |
| A | OLEAA | | 1 | 0,05 | g |

Two samples were prepared as described in table 12 and Table 13. OLEAA was diluted at 1% in paraffinum liquidum used as a solvent. The purpose of the study is to evaluate the impact of 1 wt% of OLEAA on a selection of genes expressed in the dermis and epidermis. OLEAA was applicated on 3D reconstructed Skin Full Skin Equivalent. Afterward, RNAc were collected after 48 hours of treatment and analyzed by TaqMan Low Density Arrays. These genes correspond to different biological pathways such as: autophagy, detoxification, oxidative stress response, inflammation, hydration, cell adhesion, antimicrobial defense, desquamation, dermal and epidermal biology, and lipid synthesis.

The objective was to evaluate the potential efficacies of OLEAA.

### Results of the comparison:

**Table 14: comparison of the overexpressed genes expressed by the active, only on the dermis:**

| **Formulations** | **Dermal benefits - gene** | | **Biological function** | **Fold change** | **p-value** |
|---|---|---|---|---|---|
| | **Symbol** | **Name** | | | |
| MD.02 versus MD.01 | COL7A1 | collagen 7 alpha 1 subunit | dermo-epidermal junction | 1,61 | 0,04 |
| | CSGALNACT1 | Chondroitin sulfate N-acetylgalactos-aminyltransferase 1 | Synthesis and regulation of GAGs | 1,77 | 0,00 |
| | FTH1 | Ferritin heavy chain | response to oxidative stress | 2,13 | 0,01 |
| | GPX1 | Glutathione peroxidase 1 | response to oxidative stress | 1,88 | 0,04 |
| | MT2A | Metallothionein 2A | response to oxidative stress | 1,88 | 0,01 |
| | NQO1 | NAD(H)dehydrogenase, quinone 1 | response to oxidative stress | 1,94 | 0,02 |
| | PPARD | Peroxisome proliferator-activated receptor delta | Synthesis and regulation of lipids | 1,33 | 0,02 |
| | PRDX6 | Peroxiredoxin-6 | response to oxidative stress | 1,82 | 0,02 |

| | | | | | |
|---|---|---|---|---|---|
| MD.01 corresponds to the placebo formula MD.02 corresponds to the OLEAA diluted at 1%. The impact of liquid paraffin has not been taken into account. | | | | | |

### Antioxidant response:

Overexpression of the following genes: FTH1, GPX1, MT2A, NQ01, PRDX6, PPARD.

### • FTH1 - part of the ferrithin protein:

These proteins are upregulated faced to a radical stress. The proteins are responsible of the stock of Fer molecules and help to regulate their availability.

### • GPX1 (Glutathione peroxidase 1) :

Glutathione peroxidase 1 is an antioxidant enzyme. The role of this enzyme is to face oxidative stress, to protect DNA, lipids and proteins. The overexpression of GPX1 demonstrates a lack of detoxification process.

### • MT2A (Metallothionein 2A) :

Metallothionein are small intracellular proteins. These proteins play an important role in chelating heavy metals. The induction of metallothionein is conducted by different stimuli:
heavy metals, stress, UV, hormones, and proinflammatory cytokines. Metallothionin contribute to the cellular detoxification.

### • NQO1 (NAD(H)dehydrogenase quinone 1) :

NAD(H)dehydrogenase quinone 1 is a flavoprotein overexpressed with a pro-oxidative stress. This protein plays a role in reducing the production of radical oxidative species.

### • PRDX6 (Peroxiredoxin 6) :

Peroxiredoxin-6 is part of a specific antioxidant system.

### • PPARD (Peroxisome proliferator activated receptor delta):

Peroxisome proliferation activated receptor delta plays an important role in the healing by regulating the protein of extracellular matrix, anti-inflammatory response in dermal fibroblasts and keratinocytes. This receptor decreases the secretion of MMP-1 (metalloprotein matrix 1) also known as collagenase 1 and MMP-9 (metalloprotein matrix 9).

The MMP is responsible for collagen and elastin degradation. PPAR delta also regulates the gene CAT (catalase). A well-known antioxidant enzyme.

### Conclusion:

In biological pathways, oxidative damage accumulates with age. Oxidative stress could be induced by intrinsic aging and extrinsic aging.

OLEAA demonstrates activity at different levels against oxidative stress and detoxification by the overexpression of the following genes: FTH1, GPX1, MT2A, NQO1, PRDX6, PPARD. With these results, we could say that the cosmetic composition OLEAA has a positive influence against skin aging and more preferentially on improving antioxidant response and detoxification.

The results on MT2A could announce a positive impact on the conversation of extracellular matrix.

The results on PPARD delta could be announced healing benefits.

### Epidermal dermal junction:

### Overexpression of the following genes: COL7A1

OLEAA induces a significant upregulation of the expression of Collagen 7 alpha 1.

Collagen 7 is known to play an important role in the anchorage of keratinocytes in extracellular matrix.

OLEAA could have a beneficial effect at the level of the basal membrane organization, thus ensuring a certain cohesion and a strengthening of the dermo-epidermal junction.

### Synthesis and regulation of Glucoaminoglycan

### Overexpression of the following gene: CSGALNACT1

This gene induces the synthesis of glucoaminoglycan or GAGs, essential molecules for the cohesion of the extracellular matrix in the dermis.

### General conclusion:

The topical application of OLEAA demonstrates a very interesting gene regulation in the dermis. The overexpression of these genes is implicated on the antioxidant response and detoxification (GPX1, MT2A, MQO1, PRDX6, PPARD). This regulation could favor the prevention of oxidative stress and the non-accumulation of reactive oxygen species (ROS), which could be accelerated in skin ageing. Over expression of COL7A1 could have beneficial effect on the cohesion and reinforcement of the dermal epidermal junction and ensure skin hydration and elasticity. It could be brought by CSGALNACT1 gene also.

### TEST CAT:

The CAT (Conjugated Autoxidizable Triene) test is one of the spectrophotometric tests used to assess the total antioxidant capacity of the samples. The CAT test measures a product's ability to trap free radicals, a sign of anti-aging activity. The CAT test is well suited for oil-based products. This CAT Test is well suited to oil-based products. This test assesses a product's ability to block oxidative radical reactions. It measures by UV spectrophotometry, the damage caused by free radicals on an oxidizable substrate. The presence of antioxidants in a tested product prevents free radicals from acting on the oxidizable substrate. 2,2'-azobis-2-amidinopropane dichloride (AAPH) is used to generate reactive oxygen species (ROS) and tung oil is used as substrate oxidizable. Trolox^{®} (6-hydroxy-2,5,7,8-tetramethylchromane-2-carboxylic acid), an analogue of vitamin E, is used as an external reference and the CAT results are expressed in Trolox^{®} equivalents per unit sample weight.

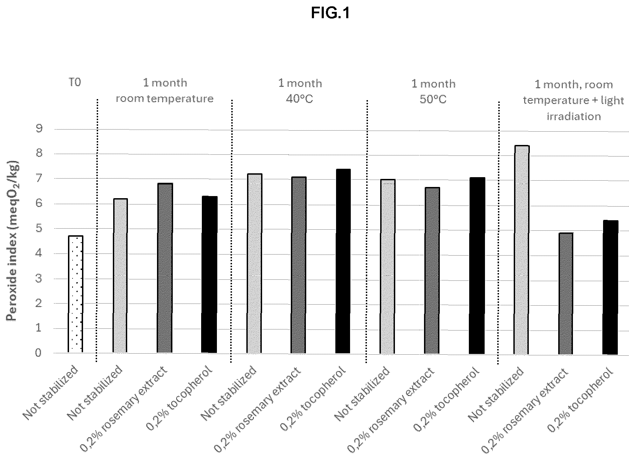

OLEAA has an improved anti-free-radical effect (see Figure 1) in comparison with virgin walnut oil

**Table 15: results of the CAT test:**

| **Batch No** | **CAT value (micromole Trolox eq/kg of product)** |
|---|---|
| OLEAA-0722.N5.FNPM.01B | 14292 |
| OLEAA-0722.N5.FNPM.02B | 15581 |
| OLEAA-0722.N5.FNPM.03B | 14734 |
| Virgin walnut oil | 0 |

### Conclusion:

These results show a powerful antioxidant activity of the active Fraxinus oleo active.

### Ex-vivo tests on protective effect of carbonylation against UV/pollution stress:

### Method :

24 explants from 30-year-old female Caucasians donors have been collected and kept alive. After reception, skin explants were distributed in 8 experimental groups (n=3 per group. Table 2), the culturing medium was renewed every 24 hours.

For "protective" efficacy evaluation, the products were topically applied overnight on the skin explants surface (2 mg/cm2). After over-night contact, an additional application of the products was performed before the stress exposure. 30 minutes after the treatments, the explants were disposed into 2 mL of Hank's Balanced Salt Solution (HBSS), topically treated with urban dust (PM10-like; Ref. ERM-CZ100; certified European Reference Material; 0,375µg/cm2; for 30 minutes of contact) and irradiated with UV-A (LED source, emission peak at λ=365 nm; 6 J/cm2; 40 minutes of irradiation) using the OxiProteomics^{®} irradiation system. The groups treated in basal conditions did not receive any application of urban dust and UVA irradiation. Two (2) hours after the irradiation, each explant was sampled, transferred in OCT for cryopreservation and snap-frozen in liquid nitrogen and conserved at -80°C until analysis.

### Dilution of OLEAA at 1 wt% and 0,1 wt%

**Table 16: placebo formulation**

| **Matter** | **INCI EU** | **% matter** |
|---|---|---|
| demineralized water | Aqua (water) | 98,4 |
| Cosmedia sp | Sodium polyacrylate | 1,0 |
| Geogard 221 | Benzyl alcohol, dehydroacetic acid, aqua | 0,6 |
| | | 100,0 |

**Table 17: formulation with 1% of OLEAA**

| **Phase** | **Matter code** | **Name matter** | **INCI EU** | **% matter** |
|---|---|---|---|---|
| A | ECH-0008 | demineralized water | Aqua (water) | 97,4 |
| A | M100505 | Cosmedia sp | Sodium polyacrylate | 1,0 |
| B | M100732 | Geogard 221 | Benzyl alcohol, dehydroacetic acid, aqua | 0,6 |
| C | ECH-3210 | OLEAA | Fraxinus excelsior bark extract, Juglans Regia (walnut) seed oil, polyglyceryl-4 oleate, tocopherol | 1,0 |
| | | | | 100,0 |

**Table 18: formulation with 0,1% of OLEAA**

| **Matter** | **INCI EU** | **% matter** |
|---|---|---|
| demineralized water | Aqua (water) | 98,3 |
| Cosmedia sp | Sodium polyacrylate | 1,0 |
| Geogard 221 | Benzyl alcohol, dehydroacetic acid, aqua | 0,6 |
| OLEAA | Fraxinus excelsior bark extract, Juglans Regia (walnut) seed oil, polyglyceryl-4 oleate, tocopherol | 0,1 |
| | | 100,0 |

**Results : Table 19: Carbonyl levels - whole skin**

| **Carbonyl levels - whole skin (see** **Figure 3****)** | | | | | | |
|---|---|---|---|---|---|---|
| **condition** | **Carbonylated proteins mean (%vs control)** | **Standard deviation** | **Protection (% vs stress)** | **Induction (% vs control)** | **p-value (vs stress)** | |
| **Basal conditions** | | | | | | |
| Control | 100 | 3 | - | - | - | - |
| OLEAA 0,1% | 103 | 16 | - | 3 | ns | 0,990 |
| OLEAA 1% | 106 | 10 | - | 6 | ns | 0,862 |
| Placebo | 106 | 4 | - | 46 | ns | 0,877 |

| **Stress conditions** | | | | | | |
|---|---|---|---|---|---|---|
| Stress (UVA irradiation) | 239 | 9 | - | - | - | - |
| OLEAA 0,1% + stress | 192 | 9 | 34 | - | *** | 0,0002 |
| OLEAA 1% + stress | 178 | 8 | 44 | - | *** | < 0,0001 |
| Placebo + stress | 226 | 12 | 9 | - | ns | 0,2899 |

| | | | | | | |
|---|---|---|---|---|---|---|
| *** : p<0.001 ; ns : not significant | | | | | | |

**Table 20:**

| **Carbonyl levels - stratum corneum (see** **Figure 4****)** | | | | | | |
|---|---|---|---|---|---|---|
| **condition** | **Carbonylated proteins mean (%vs control)** | **Standard deviation** | **Protection (% vs stress)** | **Induction (% vs control)** | **p-value (vs stress)** | |
| **Basal conditions** | | | | | | |
| Control | 100 | 18 | - | - | - | - |
| OLEAA 0,1% | 93 | 27 | - | 0 | ns | 0.987 |
| OLEAA 1% | 93 | 13 | - | 0 | ns | 0.980 |
| Placebo | 86 | 8 | - | 0 | ns | 0.857 |

| **Stress conditions** | | | | | | |
|---|---|---|---|---|---|---|
| Stress (UVA irradiation) | 260 | 33 | 0 | - | - | - |
| OLEAA 0,1% + stress | 241 | 23 | 12 | - | ns | 0,8425 |
| OLEAA 1% + stress | 202 | 23 | 36 | - | | 0,0964 |
| Placebo + stress | 246 | 27 | 8 | - | ns | 0,9474 |

**ns : not significant Table 21:**

| **Carbonyl levels - epidermis (see** **Figure 5****)** | | | | | | |
|---|---|---|---|---|---|---|
| **condition** | **Carbonylated proteins mean (%vs control)** | **Standard deviation** | **Protection (% vs stress)** | **Induction (% vs control)** | **p-value (vs stress)** | |
| **Basal conditions** | | | | | | |
| Control | 100 | 13 | - | - | - | - |
| OLEAA 0,1% | 101 | 13 | - | 1 | ns | > 0,9999 |
| OLEAA 1% | 121 | 8 | - | 21 | ns | 0,480 |
| Placebo | 115 | 6 | - | 15 | ns | 0,751 |

| **Stress conditions** | | | | | | |
|---|---|---|---|---|---|---|
| Stress (UVA irradiation) | 262 | 37 | 0 | - | - | - |
| OLEAA 0,1% + stress | 191 | 14 | 44 | - | * | 0,0136 |
| OLEAA 1% + stress | 197 | 13 | 40 | - | * | 0,0235 |
| Placebo + stress | 261 | 29 | 0 | - | ns | > 0,9999 |

| | | | | | | |
|---|---|---|---|---|---|---|
| *:p<0.05; ns : no significant | | | | | | |

**Table 22:**

| **Carbonyl levels - dermis (see** **Figure 6****)** | | | | | | |
|---|---|---|---|---|---|---|
| **condition** | **Carbonylated proteins mean (%vs control)** | **Standard deviation** | **Protection (% vs stress)** | **Induction (% vs control)** | **p-value (vs stress)** | |
| **Basal conditions** | | | | | | |
| Control | 100 | 14 | - | - | - | - |
| OLEAA 0,1% | 104 | 11 | - | 4 | ns | 0,964 |
| OLEAA 1% | 100 | 9 | - | 0 | ns | > 0,9999 |
| Placebo | 96 | 6 | - | 0 | ns | 0,977 |

| **Stress conditions** | | | | | | |
|---|---|---|---|---|---|---|
| Stress (UVA irradiation) | 210 | 9 | 0 | - | - | - |
| OLEAA 0,1% + stress | 168 | 7 | 38 | - | - | 0,0012 |
| OLEAA 1% + stress | 159 | 9 | 46 | - | *** | 0,0003 |
| Placebo + stress | 203 | 9 | 7 | - | ns | 0,7793 |

| | | | | | | |
|---|---|---|---|---|---|---|
| *** : p<0.001; ns : not significant | | | | | | |

### Conclusion:

Protein carbonylation, a final step of oxidative stress, has been associated with protein dysfunctions, cellular and tissue disorders, aging, and age-related diseases. Elevated levels of carbonylated proteins have been linked to changes in mechanical properties, hydration functions, and skin color in the skin.

Therefore, preventing protein carbonylation is crucial to protect the skin against phenotypic alterations.

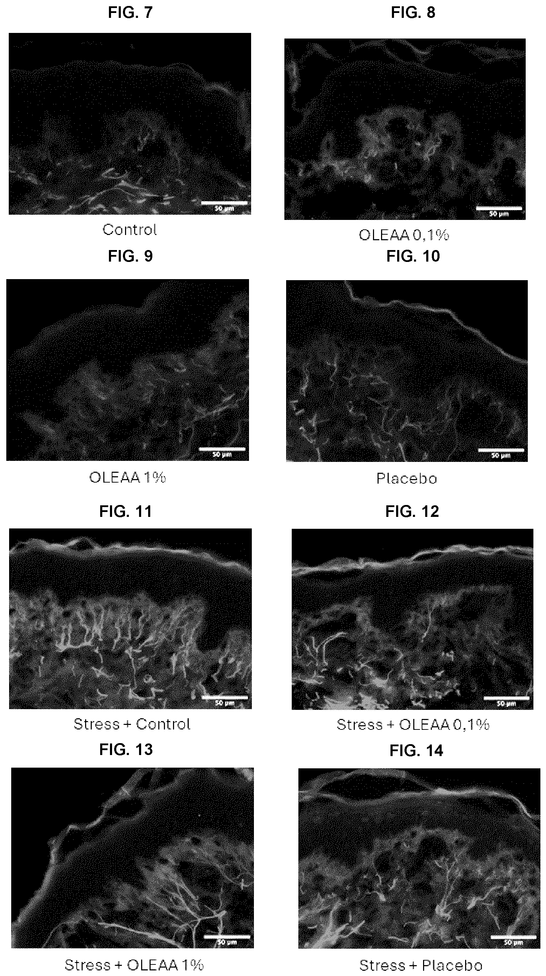

The skin explants are treated with OLEAA (1% and 0,1%) or without (Placebo). Explants are then stressed to induce carbonylation (UV and Pollution) which corresponds to the result "Stress conditions". The results show that placebo does not induce an effect on carbonylation and that the active ingredient from 0.1% significantly reduces protein carbonylation on analyses conducted on the skin in its entirety, the epidermis and the dermis. Fig. 7 to Fig.14 show some pictures of the dermis.

On the whole skin:
The active OLEAA at 0.1% has a protective effect of +34%
The active OLEAA at 1% has a protective effect of +44%
The results on the Stratum Corneum are not significant and therefore not interpretable.

On the epidermis:
The active OLEAA at 0.1% has a protective effect of +44%
The active ingredient OLEAA 1% has a protective effect of +40%

On the dermis:
The active OLEAA at 0.1% has a protective effect of +38%
The active ingredient OLEAA 1% has a protective effect of +46%

Therefore, the active OLEAA has a protective effect on the carbonylation of proteins during a stress UV + pollution and thus has an anti-aging effectiveness.

### Test ex vivo Regeneration of the stratum corneum against physical and cortisol stress (see Figures 8 and 9)

### Test Objective:

The skin explant is subjected to physical stress (scraping of the explant) as well as chemical stress (cortisol, mimicking the effects of stress) to limit its regeneration.

The objective is to analyze the effect of OLEAA on stratum corneum regeneration method: 30 explants from 30-year-old female Caucasians donors have been collected and keeping alive. After reception, skin explants were distributed in 5 experimental groups (n=6 per group. The culturing medium was renewed every 24 hours.

Once the SC removed, the skin appeared shiny. An aqueous solution of cortisol 0.001% (27,59 µM) was applied topically (30µL) on each explant of the group "stress", and a solution containing cortisol 0.001 % (27,59 µM) + the active ingredient OLEAA at 0.1% and 1% and placebo were topically applied (30µL) on the skin explants (groups "stress + active 0.1%" "stress + active 1%", and "stress + placebo"). At Day 1, 24 hours after the first application, the treatments of skin explants were re-applied for the group "stress", "stress + active 0.1%" "stress + active 1%", and "stress + placebo", by using freshly prepared solutions. These treatments were repeated for a total of 5 applications per explant (groups "stress + active 0.1%" "stress + active 1%", and "stress + placebo"). The "Control" group did not receive any treatment, except for the renewal of the culture medium. 24 hours after the 5^{th} series of explant treatments, a solution of fluorescent dye (Lucifer Yellow, 30 µL at 1 mg/mL) was added on the surface of 3 explants per group and incubated for 1 hour.

**Table 23:**

| **Stratum corneum thickness (see** **Figures 8 and 9** **)** | | | | |
|---|---|---|---|---|
| **condition** | **Mean (micrometer)** | **Standard deviation** | **Efficacy (%)** | **p-value (vs stress)** |
| Control | 25 | 1 | 100 | *** |
| Stress (stripping+ cortisol) | 8 | 1 | 0 | - |
| 0,1% OLEAA + stress | 19 | 1 | 60 | *** |
| 1% OLEAA + stress | 26 | 2 | 103 | *** |
| Placebo + stress | 8 | 0 | 1 | ns |

| | | | | |
|---|---|---|---|---|
| *** : p<0.001 ns : no significant | | | | |

### Conclusion:

We can see (Figures 8 and 9) that the active ingredient allows a better restructuring of the stratum corneum from 0.1%. We observe that the thickness of the stratum corneum is better with a 1% active treatment (+103%) than with the active treatment at 0.1% (+60%). The asset allows an improvement in thickness with both percentages. We will also remember that the placebo formula (without active) has no effect. OLEAA therefore has positive effects on the regeneration of the stratum corneum and more precisely on the thickness of the stratum corneum.

**Table 24:**

| **Number of Stratum corneum layers (see** **Figures 10 and 11****)** | | | | |
|---|---|---|---|---|
| **condition** | **Mean (Nb)** | **Standard deviation** | **Efficacy (%)** | **p-value (vs stress)** |
| Control | 10 | 1 | 100 | *** |
| Stress (stripping+ cortisol) | 3 | 1 | 0 | - |
| 0,1% OLEAA + stress | 6 | 1 | 43 | * |
| 1% OLEAA + stress | 7 | 1 | 57 | ** |
| Placebo + stress | 4 | 1 | 14 | ns |

| | | | | |
|---|---|---|---|---|
| *:p<0.05 **: p<0.01 *** : p<0.001 ns : not significant | | | | |

### Conclusion:

We can see (at Figures 10 and 11) that the active ingredient allows a better restructuring of the stratum corneum as of 0.1%. We observe that the thickness of the stratum corneum is better with a treatment at 1% (57%) of OLEAA than at 0.1% (43%).

OLEAA allows an improvement in the number of layers with both percentages.

We will also remember that the placebo formula (without active) has no effect. OLEAA therefore has positive effects on the regeneration of the stratum corneum and more precisely on the increase in the number of layers.

### Permeability of the skin barrier due to physical and chemical stress (see Figures 12 and 13):

Test objective: After treatment with active or not on explants, a fluorescent product is deposited. Fluorescence (marked in yellow) allows to translate the state of permeability of the skin barrier (see Figure 12).

**Table 25:**

| **Barrier dysfunction (Lucifer yellow penetration)** | | | | |
|---|---|---|---|---|
| **condition** | **Lucifer yellow Mean (% vs control)** | **Standard deviation** | **Efficacy (%)** | **p-value (vs stress)** |
| Control | 100 | 2 | 100 | *** |
| Stress (stripping+ cortisol) | 128 | 2 | 0 | - |
| 0,1% OLEAA + stress | 95 | 4 | 118 | *** |
| 1% OLEAA + stress | 109 | 5 | 68 | *** |
| Placebo + stress | 123 | 6 | 19 | ns |

| | | | | |
|---|---|---|---|---|
| *** : p<0.001 ns : no significant | | | | |

### Conclusion:

It is observed that yellow fluorescence decreases with the application of the active ingredient OLEAA to 0.1% and 1% compared to the stress situation or fluorescence is very important; The numerical results indicate that fluorescence is lowered with the effect of the active ingredient OLEAA to 0.1% and 1%. It will also be noted that fluorescence is higher at 1% (+68%) than at 0.1% (+118%). The active ingredient allows a decrease in the permeability of the barrier with the two %. The barrier is less permeable with the active OLEAA at 0.1%, it will also be noted that the decrease is stronger than the basal condition (Control, explant not subject to stress).

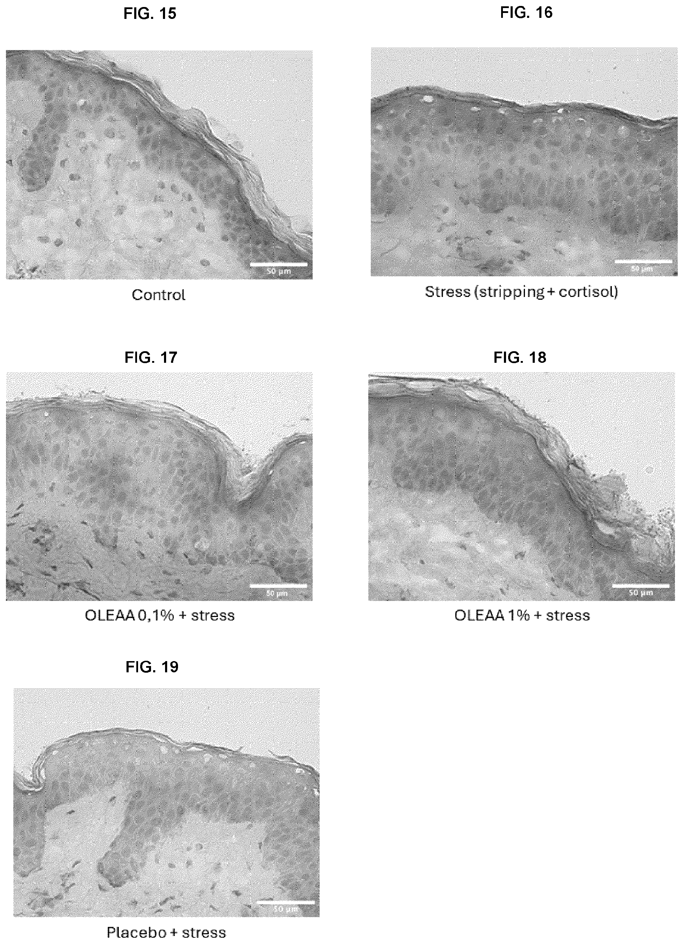

### Quantification of filaggrin levels (see Figures 14 and 15) :

Test objective: After treatment with active or not on explants, the level of filaggrin will be monitored.

Filaggrin is a precursor protein of Natural Moisturizing Factors (NMF) production of the skin and are involved in the barrier function of the skin and the hydration of the stratum corneum.

**Table 26:**

| **Filaggrin levels** | | | | |
|---|---|---|---|---|
| **Condition** | **Lucifer yellow Mean (% vs control)** | **Standard deviation** | **Efficacy (%)** | **p-value (vs stress)** |
| Control | 100 | 7 | 100 | *** |
| Stress (stripping+ cortisol) | 79 | 1 | 0 | - |
| 0,1% OLEAA + stress | 92 | 3 | 61 | ** |
| 1% OLEAA + stress | 95 | 1 | 76 | ** |
| Placebo + stress | 79 | 1 | 0 | ns |

| | | | | |
|---|---|---|---|---|
| **: p<0.01 *** : p<0.001 ns : not significant | | | | |

### Conclusion:

There is a change in the presence of filaggrin when explants are under stress. Explants treated with active has an increased rate of filaggrin and this from 0.1%

The expression of filaggrin is increased by 61 % when explants is treated with OLEAA to 0.1%. The expression of filaggrin is increased by 76% when explant is treated with 1% OLEAA. The active ingredient OLEAA boosts the presence of filaggrin in the stratum corneum following physical and chemical stress at cortisol. As a result, hydration is also boosted.

### General conclusion:

OLEAA at 0.1% and 1% shows impressive significant results on:
- Restoration of the corneocytes layers
- Restoration of stratum corneum thickness
- Restoration skin barrier integrity
- Improvement of hydration marker expression (filaggrin)

All these benefits were proven even at low concentration of OLEAA (0.1%) with the protection of the stratum corneum and restoration of skin barrier.

### Stability:

**Table 27:**

| Batch No | **OLEAA 0722.N5.FNPM.03A** | | | |
|---|---|---|---|---|
| Storage time | T0 | T6 months | | |
| Storage conditions | | 4-8°C | Room temperature | 40°C |
| Aspect/ color/ odor | Homogenous clear oily / Yellow brown to yellow green/ Grass vegetal - woody fatty fruit nut | Light deposit at the bottom of the packaging/ similar to T0 / similar to T0 | Similar to T0/ very slightly darker/ similar to T0 | Similar to T0 / very slightly darker/ similar to T0 |
| Peroxide index (meqO2/kg) | 4,8 | 5,5 | 7,9 | 19,8 |
| Content in Fraxin (mg/kg) +/- 5% | 206 | 221 | 211 | 207 |

### Conclusion:

Asset stability was monitored under various conditions: 4°C, ambient temperature, temperature +light, 40°C, 50°C.

OLEAA is stable over time and the content of Fraxin and total polyphenols remains stable.

Walnut oil is known as an unstable oil that becomes rancid very quickly.

Here in the context of OLEAA, the hypothesis is as follows:
The known antioxidant properties of Fraxin may have a role in the preservation of OLEAA globally.

### Figures of the present invention:

**Figure 1** shows the index of peroxide (meqO₂/kg), from the left to the right: T0 (value: 4,7), without antioxidant (T=1month, room T°, value: 6,2), with 0,2% of rosemary extract (T=1month, room T, value: 6,8°), with 0,2% of tocopherol (T=1month, room T°, value: 6,3), without antioxidant (T=1month, 40°C, value: 7,2), with 0,2% of rosemary extract (T=1month, 40°C, value: 7,1), with 0,2% of tocopherol (T=1month, 40°C, value: 7,4), without antioxidant (T=1month, 50°C, value: 7), with 0,2% of rosemary extract (T=1month, 50°C, value: 6,7), with 0,2% of tocopherol (T=1month, 50°C, value: 7,1), without antioxidant (T=1month, room T°+ light, value: 8,4), with 0,2% of rosemary extract (T=1month, room T°+ light, value: 4,9), with 0,2% of tocopherol (T=1month, room T°+ light, value: 5,4). Figure 1 relates to the Comparison of Peroxyde index between CO2 supercritical rosemary extract and tocopherol.

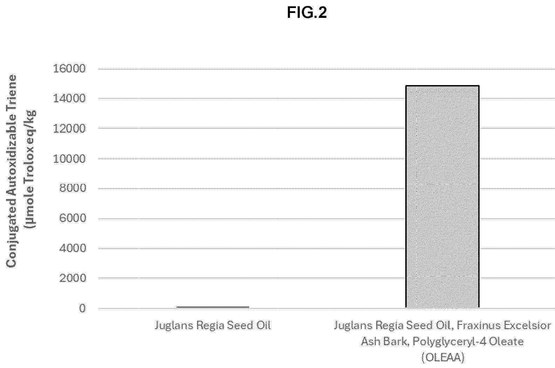

**Figure 2** shows a comparison of the anti-radical capacity between the virgin walnut oil (value 0) - the myrtle-olive oleoactive (value 1070) and the OLEAA (value 14869 micromole Trolox eq/kg of product), from the left to the right. The highest anti-radical capacity is found with OLEAA, while virgin walnut oil does not have any anti-radical capacity. Figure 1 relates to the result of CAT values (µmpole Trolox equivalent/kg of product).

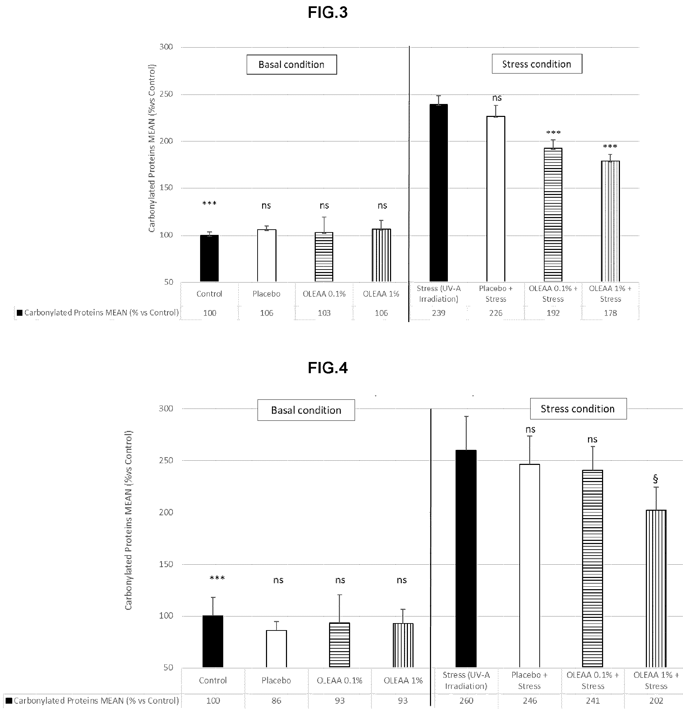

**Figure 3** shows the results of ex vivo test results (skin explants) on proteins carbonylation on the whole skin. From the left to the right: control, placebo, OLEAA 0,1%, OLEAA 1%, stress (UV-A irradiation), placebo + stress, OLEAA 0,1% + stress, OLEAA 1% + stress. Figure 2 relates to protein carbonylation levels on whole skin in basal and stress (UV-A irradiation and particulate matter) condition, ns: non-significant, ***p<0.001.
**Figure 4** shows the results of ex vivo test results (skin explants) on proteins carbonylation on the stratum corneum. From the left to the right: control, placebo, OLEAA 0,1%, OLEAA 1%, stress (UV-A irradiation), placebo + stress, OLEAA 0,1% + stress, OLEAA 1% + stress. Figure 3 relates to protein carbonylation levels on stratum corneum in basal condition and stress (UV-A irradiation and particulate matter) conditions, ns : non-significant, *p<0.1, ***p<0.001.

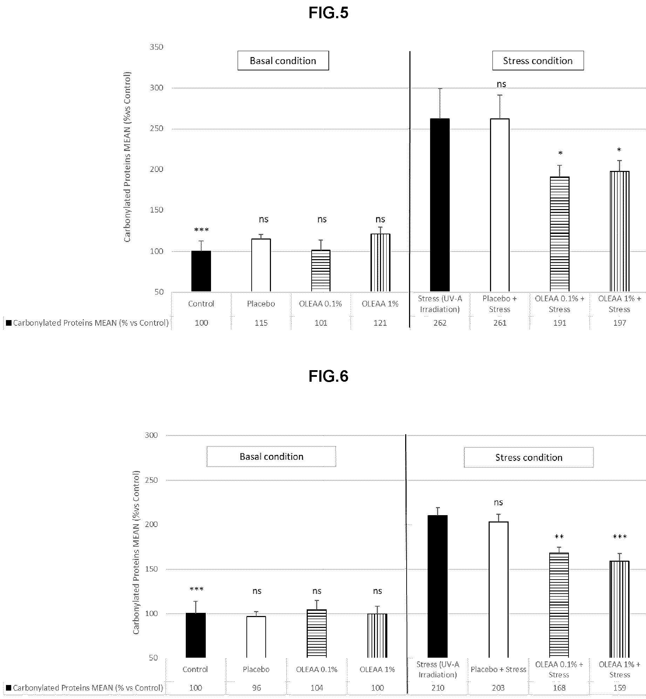

**Figure 5** shows the results of ex vivo test results (skin explants) on proteins carbonylation on the epidermis. From the left to the right: control, placebo, OLEAA 0,1%, OLEAA 1%, stress (UV-A irradiation), placebo + stress, OLEAA 0,1% + stress, OLEAA 1% + stress. Figure 4 relates to protein carbonylation levels on epidermis in basal and stress (UV-A irradiation and particulate matter) condition, ns: non-significant, * p<0.05,***p<0.001.
**Figure 6** shows the results of ex vivo test results (skin explants) on proteins carbonylation on the dermis. From the left to the right: control, placebo, OLEAA 0,1%, OLEAA 1%, stress (UV-A irradiation), placebo + stress, OLEAA 0,1% + stress, OLEAA 1% + stress. Figure 5 relates to protein carbonylation levels on dermis in basal and stress (UV-A irradiation and particulate matter) condition, ns: non-significant, **p<0.01, ***p<0.001.
**Figure 7** shows the in-situ visualization of carbonyl levels by epifluorescence microscopy of the control in basal conditions.
**Figure 8** shows the in-situ visualization of carbonyl levels by epifluorescence microscopy of OLEAA at 0,1% in basal conditions.
**Figure 9** shows the in-situ visualization of carbonyl levels by epifluorescence microscopy of OLEAA at 1% in basal conditions.
**Figure 10** shows the in-situ visualization of carbonyl levels by epifluorescence microscopy of the placebo in basal conditions.
**Figure 11** shows the in-situ visualization of carbonyl levels by epifluorescence microscopy of the control in stress conditions (UV-A and particulate matter)
**Figure 12** shows the in situ visualization of carbonyl levels by epifluorescence microscopy of OLEAA 0,1% in stress conditions (UV-A and particulate matter)
**Figure 13** shows the in situ visualization of carbonyl levels by epifluorescence microscopy of OLEAA 1% in stress conditions (UV-A and particulate matter)
**Figure 14** shows the in-situ visualization of carbonyl levels by epifluorescence microscopy of the placebo in stress conditions (UV-A and particulate matter)
   Figure 7 to Figure 14 relate to in situ visualization of carbonyl levels (grey) on basal and stress (UV-A irradiation) conditions by epifluorescence microscopy. Scale bar 50 µm.
**Figure 15** shows the in-situ visualization of the stratum corneum thickness of the control,
**Figure 16** shows the in-situ visualization of the stratum corneum thickness of the control after stress conditions (stripping+cortisol).
**Figure 17** shows the in-situ visualization of the stratum corneum thickness with OLEAA at 0,1% after stress conditions (stripping + cortisol)
**Figure 18** shows the in-situ visualization of the stratum corneum thickness with OLEAA at 1% after stress conditions (stripping + cortisol)
**Figure 19** shows the in situ visualization of the stratum corneum thickness with the placebo after stress conditions (stripping + cortisol)
   Figure 15 to Figure 19 relate to in situ visualization stratum corneum thickness under mechanical and chemical stress and OLEAA protection (0.1% and 1%).

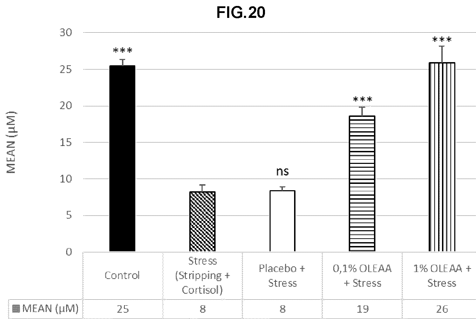

**Figure 20** shows a diagram of the stratum corneum thickness under different conditions. From the left to the right: control, stress (stripping + cortisol), placebo + stress, 0,1% OLEAA + stress, 1% OLEAA + stress.

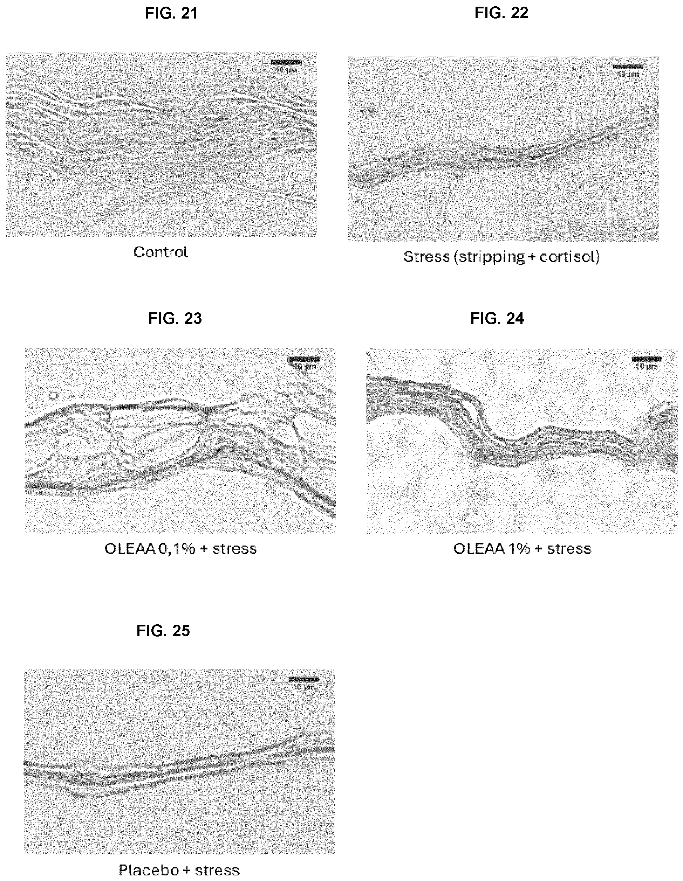

**Figure 21** shows the number of stratum corneum layers of the control.
**Figure 22** shows the number of stratum corneum layers of the control after stress conditions (stripping + cortisol).
**Figure 23** shows the number of stratum corneum layers with OLEAA at 0,1% after stress conditions (stripping + cortisol).
**Figure 24** shows the number of stratum corneum layers with OLEAA at 1% after stress conditions (stripping + cortisol).
**Figure 25** shows the number of stratum corneum layers with the placebo after stress conditions (stripping + cortisol).
Figure 21 to Figure 25 relate to in situ visualization of corneocytes layers number under mechanical and chemical stress and OLEAA protection (0.1% and 1%).

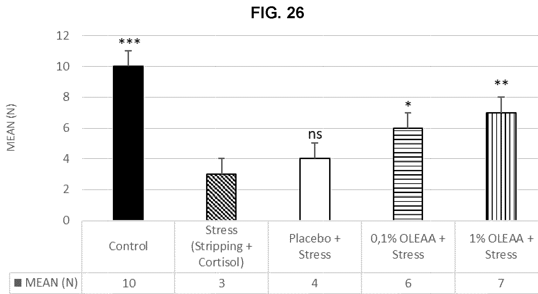

**Figure 26** shows the quantification of stratum corneum layers. From the left to the right: control, stress (stripping + cortisol), placebo + stress, 0,1% OLEAA + stress, 1% OLEAA + stress. The number of corneocyte layers of each experimental group is reported as mean +/-SD per experimental group. ***p<0,001; **p<0,01; *p<0,05; ns means not significantly different.

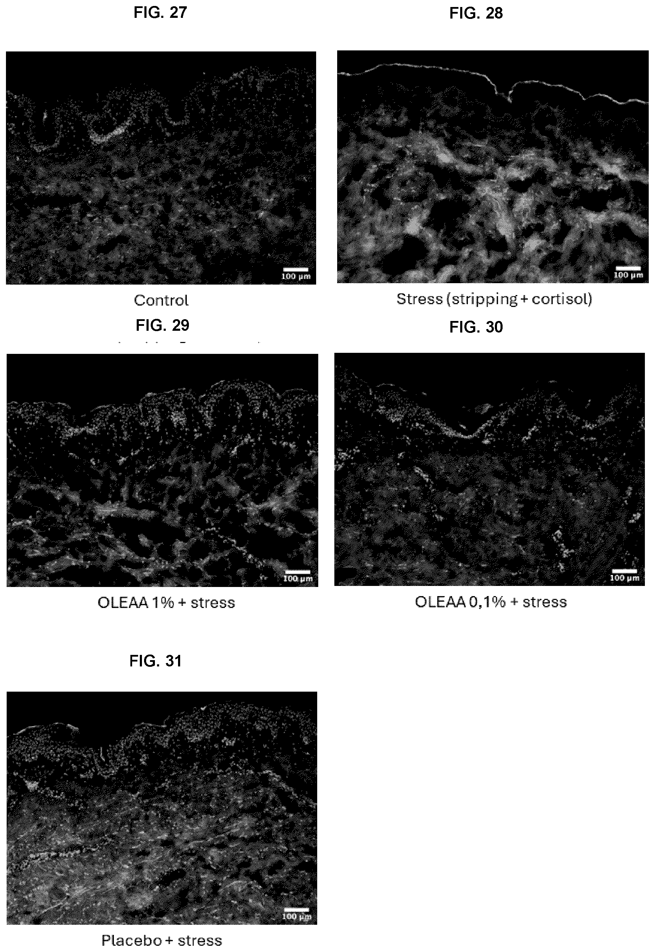

**Figure 27** shows in situ visualization of fluorescent dye permeability for skin barrier integrity evaluation of the control.
**Figure 28** shows in situ visualization of fluorescent dye permeability for skin barrier integrity evaluation of the control after stress conditions (stripping+cortisol).
**Figure 29** shows in situ visualization of fluorescent dye permeability for skin barrier integrity evaluation with OLEAA at 1% after stress conditions (stripping+cortisol).
**Figure 30** shows in situ visualization of fluorescent dye permeability for skin barrier integrity evaluation with OLEAA at 0,1% after stress conditions (stripping+cortisol).
**Figure 31** shows in situ visualization of fluorescent dye permeability for skin barrier integrity evaluation with the placebo after stress conditions (stripping+cortisol).
   Figure 27 to Figure 31 relate to in situ visualization of integrity of skin barrier under mechanical and chemical stress and OLEAA action (0.1% and 1%). The representation of the images was obtained by Lucifer Yellow penetration on skin explant surface. The specific signal of Lucifer Yellow is shown superposed to the nuclei staining (DAPI, in cyan).

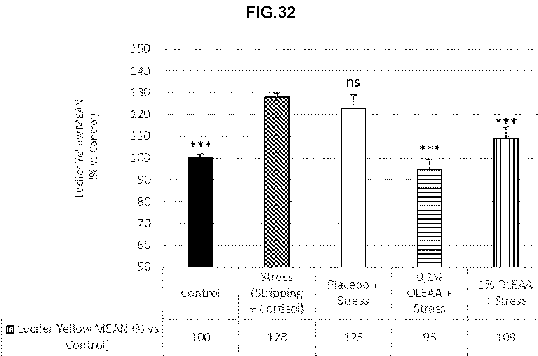

**Figure 32** shows a diagram of the quantification of fluorescent dye permeability. From the left to the right: control, stress (stripping + cortisol), placebo + stress, 0,1% OLEAA + stress, 1% OLEAA + stress.

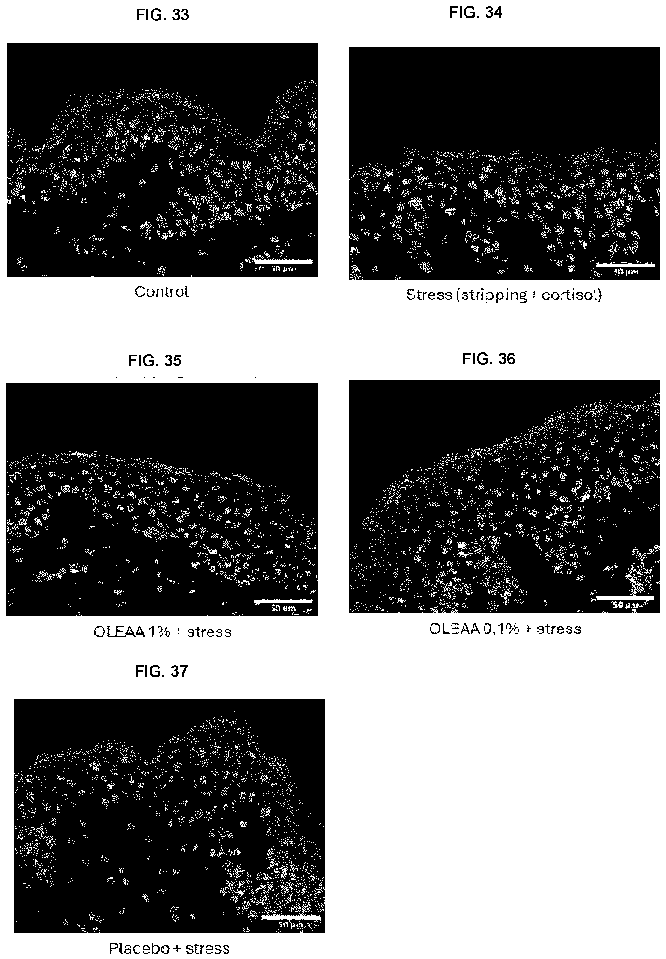

**Figure 33** shows the in-situ visualization of Filaggrin (green) levels by epifluorescence microscopy of the control.
**Figure 34** shows the in-situ visualization of Filaggrin (green) levels by epifluorescence microscopy of the control after stress conditions (stripping+cortisol).
**Figure 35** shows the in-situ visualization of Filaggrin (green) levels by epifluorescence microscopy with OLEAA at 1% after stress conditions (stripping+cortisol).
**Figure 36** shows the in-situ visualization of Filaggrin (green) levels by epifluorescence microscopy with OLEAA at 0,1% after stress conditions (stripping+cortisol).
**Figure 37** shows the in-situ visualization of Filaggrin (green) levels by epifluorescence microscopy with the placebo after stress conditions (stripping+cortisol).
**Figure 33 to Figure 37** relate to in situ visualization fillagrin expression under mechanical and chemical stress and OLEAA action (0.1% and 1%). The specific signal of Flaggrin is shown superposed to the nuclei staining (DAPI, in cyan).

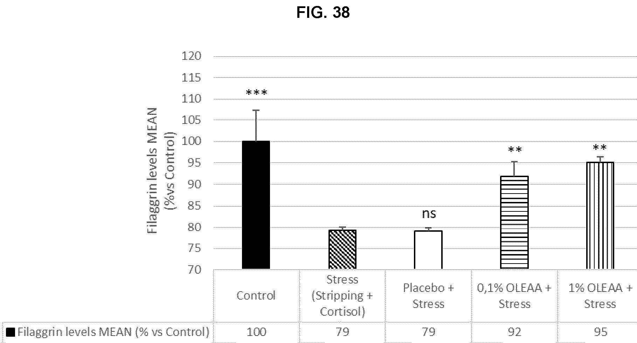

**Figure 38** shows the quantification of Fillagrin levels. From the left to the right: control, stress (stripping + cortisol), placebo + stress, 0,1% OLEAA + stress, 1% OLEAA + stress.

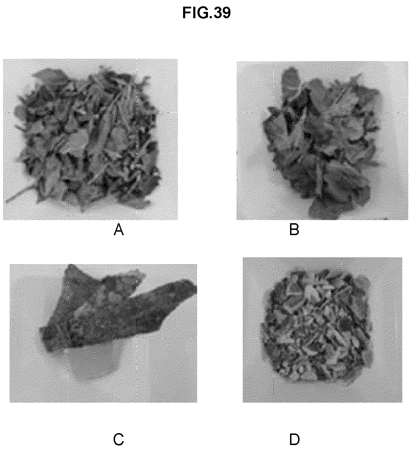

**Figure 39** shows photograph of plant parts analyzed using the Total Polyphenol Content Assay (Folin-Ciocalteu) method. A is photograph on receipt of FFREb130421-1, B is photograph on receipt of FFEb190521-1, C is photograph on receipt of EFREb040521-1, and D is photograph on receipt of EFRE280122-1.

## Claims

1. An intermediary cosmetic composition, **characterized in that** it comprises:
- (a1) 1 to 30 wt% of an extract of Fraxinus Excelsior Ash bark, and
- (b1) 60 to 80 wt% of Juglans Regia walnut seed oil, and
- (c1) 5 to 30 wt% of polyglyceryl-4 oleate, and
- (d1) 0,1 to 1 wt% of Tocopherol.

2. The intermediary cosmetic composition of claim 1, **characterized in that** it comprises:
- (a2) 5 to 20 wt% of an extract of Fraxinus Excelsior Ash bark, and
- (b2) 65 to 75 wt% of Juglans Regia walnut seed oil, and
- (c2) 10 to 25 wt% of polyglyceryl-4 oleate, and
- (d2) 0,1 to 0,5 wt% of Tocopherol.

3. The intermediary cosmetic composition of claim 1, **characterized in that** it comprises:
- (a3) 9 to 13 wt% of an extract of Fraxinus Excelsior Ash bark, and
- (b3) 69 to 73 wt% of Juglans Regia walnut seed oil, and
- (c3) 16 to 20 wt% of polyglyceryl-4 oleate, and
- (d3) 0,1 to 0,3 wt% of Tocopherol.

4. The intermediary cosmetic composition of claim 1, **characterized in that** it comprises:
- (a4) 11 wt% of an extract of Fraxinus Excelsior Ash bark, and
- (b4) 71 wt% of Juglans Regia walnut seed oil, and
- (c4) 17,78 wt% of polyglyceryl-4 oleate, and
- (d4) 0,22 wt% of Tocopherol.

5. The intermediary cosmetic composition of claim 1, **characterized in that** the extract of Fraxinus Excelsior Ash bark can be replaced by any Ash bark extract among the following list: Fraxinus apelata, Fraxinus ararica, Fraxinus atrovirens, Fraxinus anomala, Fraxinus dipetala, Fraxinus quadrangulata, Fraxinus mandshurica, Fraxinus nigra, Fraxinus platypoda, Fraxinus americana, Fraxinus Berlandieriana, Fraxinus Caroliniana, Fraxinus Latifolia, Fraxinus Papillosa, Fraxinus Pennsylvanica, Fraxinus Profunda, Fraxinus Texensis, Fraxinus Uhdei, Fraxinus Velutina, Fraxinus Apertisquamifera, Fraxinus Bungeana, Fraxinus Floribunda, Fraxinus Griffithii, Fraxinus Lanuginoa, Fraxinus Malacophylla, Fraxinus Ornus, Fraxinus Paxiana, Fraxinus Raibocarpa, Fraxinus Sieboldina, Fraxinus Trifoliolata, Fraxinus Baroniana, Fraxinus Chinensis, Fraxinus Longicuspis, Fraxinus Micrantha, Fraxinus Dubia, Fraxinus Gooddingii, Fraxinus Greggii, Fraxinus Purpusii, Fraxinus Americana L., Fraxinus angustifolia, Fraxinus pennsylvanica Marshall.

6. The intermediary cosmetic composition of claim 1, **characterized in that** the extract of Fraxinus Excelsior Ash bark comprises Fraxin, Flavaprin, Icaritin 3-rhamnoside, Machaerol B, Phloretin 2"-O-(6-O-acetylglucosid) and Ovalitenin A.

7. The intermediary cosmetic composition of claim 1, **characterized in that** Juglans Regia walnut seed oil can be replaced by any walnut seed oil among the following list: Juglans amara, Juglans illinoinensis, Juglans ovata, Juglans alba, Juglans ailantifolia, Juglans fallax, Juglans x sinensis, Juglans australis, Juglans mandshurica, Juglans nigra, Juglans venezuelensis, Juglans fraxinifolia.

8. A final cosmetic product composition, **characterized in that** it comprises:
- (a5) 0,09 to 0,25 wt% of an extract of Fraxinus Excelsior Ash bark,
- (b5) 0,69 to 1,50 wt% of Juglans Regia walnut seed oil,
- (c5) 0,16 to 0,20 wt% of polyglyceryl-4 oleate,
- (d5) 0,001 to 0,730 wt% of tocopherol,
- (e5) the rest being several other ingredients.

9. The final cosmetic product composition according to claim 8, **characterized in that** it comprises:
- (a6) 0,09 to 0,20 wt% of an extract of Fraxinus Excelsior Ash bark,
- (b6) 0,69 to 1,20 wt% of Juglans Regia walnut seed oil,
- (c6) 0,17 to 0,19 wt% of polyglyceryl-4 oleate,
- (d6) 0,001 to 0,500 wt% of tocopherol,
- (e6) the rest being several other ingredients.

10. The final cosmetic product composition according to claim 8, **characterized in that** it comprises:
(a7) 0,09 to 0,13 wt% of an extract of Fraxinus Excelsior Ash bark,
(b7) 0,69 to 0,73 wt% of Juglans Regia walnut seed oil,
(c7) 0,16 to 0,20 wt% of polyglyceryl-4 oleate,
(d7) 0,001 to 0,730 wt% of tocopherol,
(e7) the rest being several other ingredients.

11. The final cosmetic product composition according to claim 8, **characterized in that** it comprises:
- (a8) 0,22 wt% of an extract of Fraxinus Excelsior Ash bark,
- (b8) 1,42 wt% of Juglans Regia walnut seed oil,
- (c8) 0,177 wt% of polyglyceryl-4 oleate,
- (d8) 0,003 wt% of tocopherol,
- (e8) the rest being several other ingredients.

12. The final cosmetic product composition according to claim 8, **characterized in that** it comprises:
- (a9) 0,11 wt% of an extract of Fraxinus Excelsior Ash bark,
- (b9) 0,71 wt% of Juglans Regia walnut seed oil,
- (c9) 0,177 wt% of polyglyceryl-4 oleate,
- (d9) 0,003 wt% of tocopherol,
- (e9) the rest being several other ingredients.

13. The final cosmetic product composition according to any of claims 8 to 12, **characterized in that** it is applied on the skin as an emulsion, or an oil, or a serum, or a gel, or a spray, or a balm, or a lotion, or a stick, or a foam, or a shower gel.

14. A non-therapeutic cosmetic process for skin care, in particular for protecting the skin from environmental aggressions, preventing, delaying and/or fighting the signs of skin aging, consisting of applying the final cosmetic product composition of any of claims 8 to 12 to the affected areas of the skin .

15. A non-therapeutic use of the final cosmetic product composition of any of claims 8 to 12, for cosmetic protection of the skin against environmental aggressions, for cosmetic prevention, for cosmetic delaying and/or fighting the signs of skin aging, for cosmetic improvement of the skin barrier, for cosmetic improvement of the hydration ensuring a certain cohesion and a strengthening of the dermo-epidermal junction and the extracellular matrix, for cosmetic anti-inflammatory benefits, for cosmetic antioxidant and detoxification benefits, for cosmetic anti-microbial benefits and for cosmetic skin pigmentation regulation.
